# EUROPEAN PATENT APPLICATION

(11) **EP 2 172 567 A2**
(43) Date of publication of application: **07.04.2010**
(21) Application number: 09171033.5
(22) Date of filing: 22.09.2009
(51) Int. Cl.: C12Q 1/68, G01N 33/68

(54) **Methods for detecting nucleic acids in a sample**

(30) Priority: 23.09.2008 US 99515 P
(71) Applicant: Huang, Ying, San Diego CA 92129 (US); Light, James, San Diego CA 92131 (US); Mather, Elizabeth, San Diego CA 92129 (US); Weisburg, William, San Diego CA 92128 (US)
(72) Inventor: Huang, Ying, San Diego CA 92129 (US); Light, James, San Diego CA 92131 (US); Mather, Elizabeth, San Diego CA 92129 (US); Weisburg, William, San Diego CA 92128 (US)
(74) Representative: Harrison Goddard Foote

(57) **Abstract**

Systems and methods are provided for immobilizing nucleic acid amplicons on a test device. Amplicons comprising a synthetic binding unit and a detectable label are generated and immobilized at predetermined locations on a test device by specific binding interactions between the synthetic binding unit and a synthetic capture unit located at the predetermined locations. The synthetic binding unit may include a unique design such that during an amplification process, a region of the synthetic binding unit is not subject to the polymerase chain extension, and thus the amplicon remains single stranded and available for binding to the synthetic capture unit during the capture process. In certain embodiments, the synthetic binding unit and a synthetic capture unit include synthetic nucleic acid analogs that do not interact with native nucleic acids or enzymes that act thereon. In one embodiment the synthetic binding unit and synthetic capture unit includes pyranosyl RNA (pRNA).

## Description

### CROSS-REFERENCE TO RELATED APPLICATIONS

This application claims benefit of priority to U.S. Provisional Application Serial No. 61/099,515, which was filed September 23, 2008, and is incorporated herein in its entirety by reference.

### TECHNICAL FIELD OF THE DISCLOSURE

The present disclosure relates to a method and device for the identification of an analyte. In particular, the disclosure relates to a method, device, and system for the rapid detection of a nucleic acids and/or protein using non-native nucleic acid probes. More particularly, the disclosure relates to a nucleic acid detection system employing a lateral flow format.

### BACKGROUND OF THE DISCLOSURE

There has been increasing interest and efforts devoted to developing biosensor technologies for identifying biological agents and/or markers, for instance, pathogens, particularly in areas such as biological weapons and emerging disease diagnostics. Rapid, accurate, and sensitive detection of biological agents typically employ a broad-spectrum assay that is capable of discriminating between closely related markers, such as closely related microbial or viral pathogens. Nucleic acid sequences traditionally provide the most robust and phylogenetically informative signatures.

Currently, the most widely used method for the detection of nucleic acid sequences involves the use of PCR for the production of amplified nucleic acid products that may then be detected using one or more of the various assays discussed below. First, a sample, such as a biological fluid, for instance, blood, is obtained. Cells within the sample are then isolated and the DNA therein is collected. Once the DNA from the sample has been isolated and purified, it may then be amplified using a PCR protocol. The amplified DNA may then be detected, for instance, by employing a hybridization assay. Hybridization assays have been proposed for the post-PCR detection of amplified products, such as nucleic acids, because they are easily automatable (Bortolin S. Anal. Chem. 1994; 66:4302-4307).

Hybridization assays, however, require specialized instrumentation and multiple pipetting, incubation, and washing steps. These steps are performed in order to capture the amplified target nucleic acid sequence, contact the amplified sequence to a substrate containing a specific probe that recognizes the amplified target nucleic acid sequence, hybridize the amplified sequence with the specific probe, remove any excess probe, and read the generated signal so as to detect the presence of the target nucleic acid sequence.

Typical automated hybridization methods for the high throughput detection of nucleic acids include: DNA microarray, real-time PCR, capillary electrophoresis, and flow cytometry assays. Current high throughput nucleic acid chemistries and analyses techniques depend on the ability to manipulate them on a macroscopic scale by localizing particular nucleic acid species (or groups of species) at a known location on a substrate, such as in an array, for instance , in a DNA microarray for use in a nucleic acid detection assay.

A DNA microarray nucleic acid detection assay involves the parallel hybridization of a target DNA to an array of hundreds to thousands of complementary DNA capture oligonucleotides that are spotted on a surface of a substrate. One example is the Nanogen NC400^{®} microarray system, which is described generally in U.S. Patent No. 5,605,662. DNA microarray technology increases the information capacity of nucleic acid diagnostics and enables high throughput detection, in parallel, of large panels of distinct nucleic acid sequences (DNA Microarrays: A Practical Approach. Mark Schena, ed. Oxford: Oxford University Press, 1999; Microarray Biochip Technology. Mark Schena, ed. Natick, MA: Eaton Publishing, 2000; DNA Arrays: Methods and Protocols. Jang B. Rampal, ed. Totowa, NJ: Humana Press, 2001; Heller MJ. Ann. Rev. Biomed. Eng. (2002) 4:129-153).

However, typical DNA microarray technology suffers from several drawbacks. Long hybridization incubations are required for microarray assays, which increases the sample-to-answer times beyond what is acceptable for a rapid screening assay. Additionally, microarray methods employ designs that remain reliant upon fluorescent detection and supporting instrumentation, and do not address the need for low-cost, easily manufactured devices that can be used in the absence of laboratory infrastructures.

Real-time PCR, on the other hand, allows continuous monitoring of amplified fragments during PCR by a homogeneous fluorometric hybridization assay involving simultaneously amplifying and quantifying a target DNA molecule by using a fluorescent dye during PCR (polymerase chain reaction). Real-time PCR, however, requires highly specialized, expensive equipment along with costly reagents. Capillary electrophoresis may also be used to separate and detect DNA using a micro-capillary version of slab gel electrophoresis. The process also involves expensive devices and instruments. Alternatively, flow cytometry can be used for post-PCR detection of amplification products that are fluorescently labeled or have been subjected to an oligonucleotide ligation reaction and are captured on polystyrene beads (Chen IM, et al. Am. J. Clin. Pathol. 2004; 122:783-793). Flow cytometry, however, although suitable for the development of multiplex assays, also requires expensive instrumentation.

Additionally, a key need in the use of nucleic acids in all of these areas is the ability to manipulate them on a macroscopic scale by localizing particular nucleic acid species at a known location, such as in an array on a substrate. Typically, in these systems complexing reactions between two partners of a binding system which specifically recognize each other are used. These reactions are usually reversible and rely on the hydrogen bond dependent binding between complementary strands of nucleic acids.

The disadvantage of these methods is that the sequence used for the immobilization can potentially hybridize with the sequence to be immobilized, forming intramolecular secondary structures, may hybridize with another sequence to be immobilized, forming intermolecular secondary structures, or may hybridize with nucleic acids from the sample. The risk of such an unwanted or interfering interaction increases with the length of the nucleic acid(s) to be immobilized, as well as with the complexity of a sample (e.g., possible contaminating nucleic acids.

For instance, a significant economic and time disadvantage of using natural nucleic acids as immobilization agents is that a certain minimum sequence length is required to reach a practical level of stability and selectivity of the immobilization. It is typical to use 20-mers or longer oligonucleotides in order to achieve sufficient binding specificity. This results in the entire nucleic acid strand (composed of the sequence for recognizing the sample and the sequence for immobilization) becoming relatively long. As described above, the use of very long sequences can be disadvantageous as the use of long nucleic acid sequences increases the likelihood of secondary structure formation intramolecularly, and also increases the likelihood of transient or stable hybridization between multiple strands in solution.

Another disadvantage of the use of natural nucleic acids for immobilization is that the stability of duplexes of natural nucleic acids does not increase linearly in proportion to length (number of nucleotides in the sequence) over a large range, but rather approaches a limit which depends only on the relative percentage of CG to AT base pairs ("CG content"). Binding systems having a duplex stability exceeding the natural limit cannot be prepared using natural nucleic acids. This limitation is also problematic when applying various stringency conditions to the nucleic acid at its immobilized location: the immobilizing nucleic acid tags will also be subjected to the same stringency conditions (i.e., chaotropic agents, thermal conditions, or electrostatic forces), and may dissociate. See G. Michael Blackburn and Michael J. Gait, eds. Nucleic Acids in Chemistry and Biology, 2nd ed., 1996, Oxford University Press, New York.

A further disadvantage of using natural systems for the immobilization of nucleic acids is that such systems can be easily degraded or destroyed during their use, for instance, by degradation via contact with various enzymatic components present in the sample. For example, the technologies for the preparation or amplification of a sample nucleic acid typically employs a diverse variety of enzymes that may modify the nucleic acids to be amplified.

There is, therefore, a need for a relatively cost-effective, highly sensitive, and efficient method for the identification of an analyte in a sample that does not suffer from many of the drawbacks associated with the typical high throughput hybridization assays currently available. The present disclosure provides a method, device, and/or system that meets these and other such needs.

### SUMMARY OF THE DISCLOSURE

An aspect of the present disclosure relates to methods for the identification of an analyte, such as an analyte present in a biological sample obtained from a subject. In one instance, the method is directed to the rapid detection of one or more nucleic acids. The method may include the provision of an amplicon(s) of the one or more nucleic acids to be detected. The amplicon may be an amplicon generated using a first, e.g., forward, primer conjugated to a synthetic binding unit and a second primer, reverse primer, conjugated to a detectable moiety. The method may further include the provision of a substrate, wherein the surface thereof includes a synthetic capture unit that has been immobilized at a predetermined location thereon. The synthetic capture unit may be such that it selectively and reversibly binds the synthetic binding unit. Additionally, the method may include the contacting of the surface of the substrate with the amplicon under conditions sufficient for the amplicon to become immobilized at the predetermined location by binding of the synthetic capture unit with the synthetic binding unit. The binding of the synthetic capture unit with the synthetic binding unit produces a synthetic addressable complex. Once the synthetic capture unit has bound to the synthetic binding unit, the detectable moiety associated with the amplicon may then be detected thereby indicating the presence of the nucleic acid in the sample.

In another aspect, the present disclosure relates to devices for use in the identification of an analyte, such as an analyte present in a biological sample obtained from a subject. In one instance, the device is a test device that includes a body; a substrate, and/or an indication window. For instance, in one embodiment, the substrate may include a surface that contains one or more synthetic capture units, such as a synthetic capture unit that is capable of specifically binding with a synthetic binding unit conjugated to an amplicon of a target analyte. For example, the substrate may include a plurality of immobilized synthetic capture units that are positioned on the substrate so as to form one or more addressable lines.

In one instance, the substrate may be a membrane, such as a lateral flow membrane. The membrane may be associated with one or more of a sample pad and/or an absorbent pad, and may also be positioned such that the membrane is exposed through one or more of the windows in the body of the test device. For example, the sample pad may include an absorbent material and may be positioned downstream of the one or more windows, and the absorbent pad may include a wicking material and may be positioned upstream of the one or more windows. Specifically, in one instance, the test device includes a substrate, such as a lateral flow membrane that includes a test surface having a test pad adjacent a sample pad, as well as an absorbent pad adjacent to the test pad, wherein the adjacent pads are fluidably coupled. Accordingly, in such an instance, the sample pad of the test surface may be configured for being contacted by a fluid comprising an amplicon, as disclosed herein, and the absorbent pad may be configured for drawing the fluid toward the absorbent pad and across the test pad, e.g., via capillary action. Additionally, in some instances, as described in greater detail below, the test pad may be nitrocellulose and may include a connecting agent, such as protein (e.g., an immunoglobulin) that is conjugated to a synthetic capture agent, such as a p-RNA sequence, for the immobilization of the synthetic capture agent to the surface of the test pad. In certain instances, the synthetic capture unit is immobilized along predetermined test lines or test spots on the test pad surface.

Accordingly, in one instance, the present disclosure relates to a lateral flow test device that is feasible for point of care (POC) applications, which test device may be employed in a method for the detection of a nucleic acid. The methods and devices of the present disclosure have many advantages over other nucleic acid detection systems, such as the following advantages: (a) the methods and devices of the present disclosure utilize non-native capture units, such as pRNA based capture units, and binding reagents for immobilizing a nucleic acid sequence to be detected on a test strip; (b) workflow is simplified as no post-PCR amplification procedures are needed; (c) using the device of the disclosure the method can be accomplished rapidly (e.g., approximately 15 minute incubation time); and (d) the method and device operate at a high sensitivity, such as a sensitivity of about 0.005 ng/µL or lower, which is 50-fold more sensitive than microarrays.

The methods and devices of the disclosure contemplate a method and/or a device that employs one or more specific complementary binding units. A specific complementary binding unit may be an addressable complex that includes both a synthetic capture unit and a synthetic binding unit, for instance, where the synthetic capture unit is capable of specifically binding with the synthetic binding unit. Any specific complementary addressable units may be employed, such as those including pyranosyl RNA (pRNA), 5'-5' inverted DNA, and 2'-O'methylated oligonucleotides-based synthetic addressable binding systems. A unifying feature of the components of the addressable complex, e.g., one or more of the synthetic capture unit and/or binding units, is that during the amplification process, the SBU region is not subject to the polymerase chain extension and thus remain single stranded and available for binding to the SCU during the capture process.

For instance, in one embodiment, the system employs a non-native, synthetic pRNA binding pairs wherein each member of the binding pair system, i.e., the synthetic capture and synthetic binding units, does not bind to native furanosyl-based nucleic acids. In this manner, because of the inability of the binding pair units to bind native nucleic acids, a highly sensitive nucleic acid detection system is provided. According to the disclosure, the term "native nucleic acid" is used to denote a furanosyl-based nucleic acid.

Additionally, in another embodiment, the system may include binding pair agents that include natural or native nucleic acid sequence portions, however, due to the nature of the primers employed in the amplification reaction, only an amplicon of the nucleic acid sequence of interest is amplified. In this instance, the problem of non-specific binding in the performance of the diagnostic assay is diminished because to the extent that an amplification product is produced, the amplified product is representative of the nucleic acid sequence of interest being detected.

For example, in one embodiment, a 5'-5' inverted DNA system may be employed. In such a system a novel, chimeric oligonucleotide primer may be used in the amplification reaction so as to produce a novel synthetic binding agent. Specifically, in such a system a primer including a 5'-5' joint may be designed and used. Accordingly, in certain instances, the primer may include 3'-5' nucleic acid sequence that is combined with a 5'-3' nucleic acid sequence to produce a chimeric oligonucleotide that includes a 5'-5' join segment.

The 3'-5' nucleic acid sequence portion includes a sequence of nucleic acids that does not recognize a corresponding segment in the target nucleic acid sequence to be amplified, but does recognize a corresponding, e.g., complementary, sequence in the synthetic capture unit, thus, the 3'-5' nucleic acid sequence portion acts as a synthetic binding unit, in the context of the present disclosure. The 5'-3' nucleic acid sequence includes a portion of nucleic acid sequences that recognizes a sequence in the target nucleic acid sequence to be amplified and, thus, the 5'-3' nucleic acid sequence portion functions, in the context of the present disclosure, as a primer, e.g., a forward primer, capable of initiating transcription, and therefore, amplification of the target nucleic acid sequence. Additionally included in this configuration of the amplification system of the present disclosure is a reverse primer that is attached to a detectable label moiety. A unique feature of this system is that because the amplified strand includes the 3'-5' nucleic acid sequence portion, it is incapable of being amplified and, therefore, only the target strand of interest is amplified and available for detection in accordance with the methods and devices of the system.. In this manner, a highly sensitive nucleic acid detection system is provided.

Further, in another embodiment, the system may include a 2'-O'methylated oligonucleotides-based synthetic addressable binding system. In this system, the binding pair agents employed may bind to natural or native nucleic acid sequence portions, however, due to a chemical modification of the underlying nucleic acid structure of the primer employed in producing the amplicon, only an amplicon of the nucleic acid sequence of interest that is to be detected is amplified. In this instance, the problem of non-specific binding in the performance of the diagnostic assay is diminished because to the extent that an amplification product is produced, the amplified product is representative of the nucleic acid sequence being detected.

For example, this system may include a chimeric 2'-O-methylated RNA-DNA oligonucleotide that may be employed as a primer, e.g., a forward primer. Specifically, a non-natural RNA sequence, e.g., synthetic RNA sequence, containing methyl groups substituted for the endogenous hydrogen on the 2' position of the ribose moiety may be incorporated into the primer. In such an instance, the nucleic acid sequence may be configured such that the DNA sequence of the chimeric primer recognizes a corresponding, e.g., complementary, sequence in the target nucleic acid, and thus, the synthetic oligonucleotide is capable of hybridizing with the target nucleic acid sequence, and therefore, in the presence of a DNA polymerase capable of initiating polymerization, and thus amplification, of the target nucleic acid sequence. However, because of the methyl-substitution, the amplification product is not recognized by polymerases, and thus is not capable of being amplified efficiently. It remains single-stranded and is capable of binding to the synthetic capture unit. Additionally included in this configuration of the amplification system of the present disclosure is a reverse primer that is attached to a detectable label moiety. Another feature of this system is the fact that the methyl substitution also acts to stabilize the oligonucleotide sequence. In this manner, a highly sensitive nucleic acid detection system is provided.

Accordingly, in view of the above, in some embodiments, the specific complementary addressable complex includes a synthetic capture unit and a synthetic binding unit, wherein the synthetic capture unit and the synthetic binding unit may include complementary pyranosyl RNA (pRNA) sequences, complementary oligonucleotide analog sequences with internal 5'-5' internucleotide linkages, or complementary 2'-O-methyl oligonucleotide sequences. As set forth above, a feature of these systems is that during the amplification process, a portion of the extending amplicon (e.g., the SBU) includes a region is not subject to the polymerase chain extension, and thus remains single stranded and available for binding to the SCU during the capture process.

In some embodiments, the methods include the step of amplifying a target nucleic acid sequence to be detected, prior to carrying out the detection steps. In certain instances, the amplification process may employ a first primer, such as a primer conjugated to a first pyranosyl-RNA (p-RNA) sequence, and a second primer, such as a primer conjugated to a detectable moiety. The first and second primers may include a forward and a reverse primer. Additionally, one or more binding agents may also be included, such as complementary binding agents that are capable of binding to one another, or a binding agent that is conjugated to a detectable moiety. For instance, in certain instances, at least two complementary binding agents may be provided, wherein one or more of the first and second primers may be conjugated to a first binding agent, and a second binding agent may be conjugated to a detectable moiety. For example, in one instance, the first binding agent may be streptavidin, and the second binding agent may be biotin, wherein either the streptavidin or biotin may be conjugated to a detectable moiety, such as a fluorophore, chromophore, metal, quantum dot, or combination thereof. In certain instances, the detectable moiety may be a label such as a europium bead. In one instance, the second primer may be directly conjugated to the detectable moiety, e.g., europium bead.. The step of amplifying the nucleic acid may include a thermal cycling amplification reaction, such as a polymerase chain reaction (PCR).

In certain embodiments, the method also includes scanning the test device, e.g., a lateral flow test strip within the test device, with a detection apparatus for detecting the presence of the nucleic acid by detecting the presence of the detectable moiety at the predetermined location.

Accordingly, in certain instances, the disclosure is directed to a method for the rapid detection of one or more nucleic acids. An exemplary method may include providing a test device that includes a sample pad adjacent to a test pad, wherein a sample contacted with the sample pad is capable of flowing to the test pad. The test pad includes an SCU p-RNA sequence immobilized at a predetermined location thereon. Providing a fluid sample containing a target nucleic acid to be detected. Optionally, amplifying the target nucleic acid to produce an amplicon, which amplicon includes an SBU p-RNA sequence and may include a detectable moiety, and applying the sample comprising the amplicon to the sample pad under conditions allowing the lateral flow of the fluidic sample to the test surface in such a manner that the target nucleic acid sequence becomes immobilized at the predetermined location on the test pad by hybridization between the SBU p-RNA sequence and the SCU p-RNA sequence. The method may further include detecting the presence of the target nucleic acid by detecting the presence of a detectable moiety at the predetermined location, for instance, the detection may include using a UV light for scanning the test pad. In some embodiments, an absorbent pad is adjacent to the test surface, wherein the absorbent pad is distal to the sample pad. The sample pad, test pad, and optional absorbent pad may comprise a lateral flow test strip.

For instance, as set forth above, in certain embodiments, the disclosure provides a test device that includes a lateral flow strip. The lateral flow strip may include a lateral flow membrane positioned within the body of the test device a portion of which strip may be exposed through a single or a plurality of windows in the body. The lateral flow strip may additionally include an absorbent pad, containing a wicking material, which absorbent pad may be positioned upstream of the plurality of windows. The lateral flow strip may further include a sample pad , containing an absorbent material, which sample pad is positioned downstream of the plurality of windows. The lateral flow membrane may additionally include a plurality of addressable lines, wherein each line may have immobilized thereto a synthetic unit, such as a synthetic capture unit that is capable of specifically binding with a synthetic binding unit, such as a synthetic binding unit that is conjugated to a target analyte. The synthetic capture unit and the synthetic binding unit include complementary sequences selected from the group consisting of: pRNA sequences, 2'-O-methyl oligonucleotide sequences and 5'-5' inverted DNA sequences. The lateral flow strip is configured so as to be capable of being scanned by a detection apparatus.

In some embodiments the target analyte is an infectious agent. For instance, the infectious agent may be derived from a virus, a bacteria, a parasite, and the like. For example, the infectious agent may be a virus such as an influenza virus, HIV, hepatitis virus, adenovirus, enterovirus, parainfluenza virus, or the like. For example, the infectious agent may be a bacteria such as *Streptococcus pneumoniae, Staphylococcus aureus, Bordetella pertussis, Mycoplasma pneumoniae* or the like. For example, the infectious agent may be a fungus such as *Coccidioides immitis* and the like.

In some embodiments, the test device may include an identifying marker such as a barcode. In some embodiments, the test device has control spots or control lines for binding a control reagent for standardization.

In one aspect, the disclosure relates to a method for simultaneously detecting a nucleic acid and a protein, such as an antigen, in a sample. In certain instances, the method includes forming a mixture by mixing a sample being tested for the presence of at least one target agent, such as an antigen, with a solution containing a plurality of reagents. For instance, the solution may include an antibody-synthetic binding unit conjugate. For example, the antibody-synthetic binding unit conjugate may include an antibody that specifically binds a target antigen as well as a synthetic binding unit that specifically binds a synthetic capture unit. The solution may also include a labeled antibody, such as an antibody that specifically binds the same target antigen. The method may additionally include the step of providing an amplicon solution of a target nucleic acid from the sample. For instance, the amplicon solution may include an amplicon generated using a first primer conjugated to a synthetic binding unit and a second primer conjugated to a detectable moiety. The method may further include providing a substrate, such as a lateral flow membrane test surface, as described above, wherein the test surface includes one or more, e.g., a plurality of synthetic capture units that have been immobilized at a predetermined location on the test surface, for instance, a synthetic capture unit configured and employed for recognizing and immobilizing the protein to be detected as well as a synthetic capture unit configured and employed for recognizing and immobilizing the nucleic acid amplicon to be detected. The synthetic capture unit(s) may be such that they selectively and reversibly bind the synthetic binding unit(s), but in some instances, e.g., with respect to the detection of nucleic acids, the synthetic capture unit or the synthetic binding unit do not bind native nucleic acid sequences. Additionally, the method may include applying the mixture and the amplicon solution to the substrate, e.g., lateral flow membrane, containing a plurality of detection regions, wherein each of the detection regions include immobilized thereto a synthetic capture unit. The method may then involve flowing the mixture and the amplicon solution across the membrane, whereby the synthetic capture unit captures a complex having a synthetic binding unit to which it is directed; and detecting one or more labels in at least one of the detection regions, whereby detection of the label indicates the presence of a target agent, such as an antigen, or a target nucleic acid, in the sample.

As described above, in accordance with this embodiment, the synthetic capture unit and the synthetic binding unit can be complementary pRNA sequences. Additionally, the detectable label may include europium, such as an europium bead conjugated with a binding partner, such as streptavidin and the amplicon, or the antigenantibody may be conjugated to biotin.

In one aspect, the disclosure relates to a system. The system may include a test device, as described above, and a reader, such as a reader that reads a signal generated at any addressable line of the test device, e.g., emitted from a detectable reagent. The system may also include a machine readable encoded instructions capable of directing the reader to detect the signal or signals. The detection reagent can be labeled with a fluorophore, chromophore, metal, quantum dot, or a combination thereof, such as lanthanide or europium.

Accordingly, in one instance, the disclosure is directed to a system for the detection of one or more labeled amplicons conjugated to a synthetic binding unit. The system may include a lateral flow test strip comprising one or more discrete bands containing a specific synthetic capture unit that is capable of binding one or more amplicons conjugated to a synthetic binding unit. The system may also include a fluorescence reader that is capable of distinguishing a fluorescent signal from each of the one or more bands on the test strip, wherein the reader includes a light source, such as a light emitting diode (e.g., a light emitting diode that emits light in the UV region of the spectrum), and a photodiode capable of detecting an emitted fluorescent signal. In certain instances, the fluorescent signal is generated by europium.

In one aspect, the disclosure relates to a kit. For instance, a kit that may include one or more of the test device, reader, label, such as streptavidin-coated europeum bead, and/or reagents, as described herein, for practicing the disclosed methods of the system.

The present aspects and other objects, features, and advantages of the present disclosure will further become apparent in the following Detailed Description of the Disclosure and the accompanying Figures and embodiments.

### BRIEF DESCRIPTION OF THE FIGURES

Figure 1 shows an assay format according to the disclosure.

Figure 2 shows a typical work flow according to the disclosure.

Figure 3A shows from the reaction with a control oligonucleotide pair and the results are summarized in Figure 3B.

Figure 4A shows results of the detection method using FA amplicons generated by reverse transcriptase PCR. The results are summarized in Figure 4B.

Figure 5A shows the results from the reaction with the amplicon and SA-Eu beads under the same or different buffer conditions. The results are summarized in Figure 5B.

Figure 6A-6C shows detection sensitivity for FA amplicons under conditions of (Amplicon in 1xPCR and SA-Eu beads in LF2 for both Flu strip and BSA strip. The results are shown in Figure 6A. The results are summarized in Figure 6B and graphically illustrated in Figure 6C.

### DETAILED DESCRIPTION OF THE DISCLSOURE

The present disclosure provides novel methods, devices, and systems for the detection of an analyte, for instance, an analyte isolated from a fluid sample, such as a biological fluid sample. In another aspect, the present disclosure describes a device, such as a test device including a lateral flow membrane, and a method of using the same for detecting nucleic acids, e.g., using a lateral flow format. Accordingly, in one aspect, the disclosure concerns a method for detecting an analyte in a sample using analyte-specific conjugates. Thus, the present disclosure additionally provides new conjugates and reagent kits for use in the same.

In certain embodiments, the methods and devices herein disclosed may utilize proprietary technology directed to the use of a pair of unique synthetic nucleotide polymers, which may be a pRNA binding pair, that together form an addressable binding complex, one or more of which may include a detectable moiety, such as an europium bead, that may be employed to detect a target nucleic acid, for instance, in a lateral flow format. The pRNA addressable binding complex may include a pRNA capture unit and a pRNA binding unit, wherein the two pRNA units are capable of binding to one another, but in this instance, are not capable of binding to a natural, e.g., native, nucleic acid sequence.

Therefore, in one exemplary embodiment, the methods and devices of the subject disclosure may employ a lateral flow test strip that may include one or more of three different materials including: a sample pad, a nitrocellulose (NC) membrane, and an absorbent pad. In various instances, a capture unit, such as a pRNA capture unit, may be coupled to a protein and spotted onto the NC membrane forming a specific test line(s). Amplicons, such as PCR amplicons, may be generated using a pair of modified primers, of which one primer may be conjugated with a binding unit, such as a pRNA binding unit (e.g., a pRNA unit that is complementary to the pRNA capture unit on the NC membrane) and the other primer may be associated with a binding element, such as a part of a protein binding pair, for instance, biotin (e.g., the primer may be biotinylated). A test device, as described herein, including the lateral flow test strip(s) may be contacted with, e.g., dipped into, a sample containing one or more amplicons and a labeled binding element, such as the other member of the protein binding pair, for instance, streptavidin, which has been associated with a suitable label, e.g., a coated europium bead. The test device may be incubated in the sample, for instance, at room temperature for about 15 minutes or more. The presence of amplicons on the membrane of the lateral flow test strip, representative of the presence of the target nucleic acid sequence in the sample, may then be detected, e.g., under UV light, for instance, through the bridging between the addressable binding pair, e.g., pRNA binding pair, and the presence of the europium bead, which leads to an accumulation of fluorescent europium beads at the specific test line. Using influenza A as a model target, the detection of limit of the present methods is highly sensitive, for instance about 0.005ng/µL (=30 amol/µL), for which sensitivity is 50x greater than NC400 for influenza A.

Accordingly, a method for detecting the presence of an analyte in a sample are presented first, followed by a description of a device for use in the presented method. Systems and kits for use in practicing the disclosed methods and devices are described.

### Methods for Detecting the Presence of an Analyte in a Sample

As set forth above, in one aspect, the present disclosure concerns the detection of an analyte in a sample. The analyte may be any analyte of interest, but in some instances, the analyte is one or more of a nucleic acid, a peptide, a protein, or the like. In certain embodiments, the analyte may be derived from an infectious and/or pathogenic agent, such as an algae, a fungus, a yeast, a bacteria, a parasite, a virus, and the like. For instance, the analyte may be derived from a virus, such as an influenza virus, a HIV, herpes virus, hepatitis virus, adenovirus, enterovirus, parainfluenza virus, or the like.

In certain embodiments, the analyte may represent a marker present in a biological sample, for instance, a marker present in a biological fluid of the subject from which the sample is obtained. Where the marker is a nucleic acid the marker may be any suitable form of single- or double-stranded nucleic acid, such as a ssRNA, dsRNA, DNA, aptamer, or the like. Where the marker is a protein the marker may be any suitable form of protein, such as an antigen, antibody or fragment thereof, structural protein, epitope, or the like.

A feature of the methods and devices of the present disclosure is that they employ the generation and use of a synthetic addressable binding pair system in the detection of an analyte of interest. The synthetic addressable binding pair system include specific complementary binding pair units. The partners or units of the synthetic addressable binding pair system, as employed herein, are denoted as a synthetic binding unit (SBU) and a synthetic capture unit (SCU). The specific complementary binding pair units form an addressable complex, which addressable complex includes both the synthetic capture unit (SCU) and the synthetic binding unit (SBU), for instance, where the synthetic capture unit is capable of specifically binding with the synthetic binding unit.

In certain instances, the synthetic addressable binding pair systems disclosed herein employ modified RNAs and/or DNAs that function as paired binding agents for the immobilization and detection of the target analyte. For instance, in certain embodiments, the synthetic binding pair system may employ binding partners that include complementary pyranosyl-RNA (pRNA) or pyranosyl-DNA (pDNA) strands. The pRNA and/or pDNA binding partners are useful because they are configured such that they are not sterically capable of pairing with native, e.g., natural, nucleic acids, and further are not responsive as substrates of standard nucleic acid enzymes. Examples of other non-natural oligonucleotides and polynucleotides are the chemically modified derivatives of RNA and DNA, such as for example their phosphorothioates, phosphorodithioates, methylphosphonates, 2'-O-methyl RNA, 2'-fluoro RNA.

Accordingly, any suitable, specific complementary binding pair units may be employed, such as those including pRNA, 5'-5' inverted DNA, and 2'-O-methyl oligonucleotides-based synthetic addressable binding system, as described above.. In some embodiments, the specific complementary addressable complex includes a synthetic capture unit and a synthetic binding unit, wherein the synthetic capture unit and the synthetic binding unit may include complementary pyranosyl RNA (pRNA) sequences, or the addressable complex may include a synthetic binding unit wherein the binding unit includes a nucleic acid sequence that includes 5'-5' joint region, or the addressable complex may include complementary 2'-O-methyl oligonucleotide sequences. Methods for designing, making and using synthetic binding pair systems, in addition to those employed herein, are disclosed in U.S. Patent Publication No. 2005/0208576 (incorporated herein in its entirety by reference), which disclosure sets forth a method for conjugating finished, pre-synthesized nucleic acids with synthetic binding systems. See, for instance, the more structurally different molecules that can pair with RNA and DNA that include locked nucleic acids (LNA) or peptide nucleic acids (PNA), as set forth in: Sanghvi, Y. S., Cook, P.D., Carbohydrate Modification in Antisense Research, American Chemical Society, Washington 1994; Uhlmann; Peyman; Chemical Abstracts 90(4), 543-584 (1990), herein incorporated by reference in its entirety.

As described above, in certain instances, such as when the binding pair system employs complementary pRNAs, a characteristic of the binding pair system, as employed herein, is that the system includes modified nucleic acid sequences that are of non-natural origin, and therefore do not mimic the spatial relationship of native, e.g., natural, nucleic-acid-hybridizing structures. Thus, the SCU/SBU combinations used in the present disclosure may be prepared synthetically, and consequently may have the advantage of not interacting with natural nucleic acids. Another advantage is that the SCUs/SBUs may be configured to form more stable complexes than their nucleic acid counterparts, which are of natural origin. For instance, the binding pair units employed herein are less prone to enzymatic degradation, e.g., which may result from the contact of with various enzymes that may be present in the sample . Since the synthetic binding systems of the present disclosure do not pair with, or hybridize or bind to natural nucleic acids, oligonucleotides, or polynucleotides, the use of these systems for sorting and immobilizing nucleic acids does not lead to unwanted interaction or interference by the immobilization component, e.g., the SBC, with the nucleic acids (NA) to be detected, other biomolecules, or other components of the sample

As used herein, the terms "natural" or "native" as used in reference to "nucleic acid," as used herein, includes nucleic acids, oligonucleotides, polynucleotides, and other molecules that are capable of specifically hybridizing to their complement (or partial complement), and which are composed of natural or modified nucleotides. Molecules regarded as nucleic acids, oligonucleotides, or polynucleotides are all oligomers and polymers which occur naturally or else can be prepared synthetically and which have the ability to hybridize with oligomers of naturally occurring nucleic acids. A key characteristic of all nucleic acids, oligonucleotides, and polynucleotides, as defined herein, is their ability to pair with or bind to the naturally occurring nucleic acids.

Typically, nucleic acids have a somewhat chemically repetitive structure made up from monomeric recognition nitrogen heterocyclic base units linked through a backbone, usually furanosyl sugar with phosphodiester bridges in naturally occurring nucleic acids. Nucleic acids, oligonucleotides, and polynucleotides normally have a linear polymeric structure, but branched nucleic acid structures have been devised using chemical modifications and various branching moieties during chemical synthesis. Examples of naturally occurring nucleic acids are DNA and RNA, in which the nucleoside monomers comprising 2-deoxy-D-ribose and D-ribose, respectively. In DNA and RNA, the sugars are both in furanose form, and are joined via phosphodiester bonds to form the backbone of the polymer. The N-glycoside linked nitrogen heterocyclic bases form the specific recognition structure which allows DNA and RNA to specifically pair based on the sequence of the bases. In contrast, the term "modified" as used in reference to "nucleic acid" means a modified nucleic acid sequence, such as a synthetic nucleic acid sequence, that is capable of binding a complementary modified, e.g., synthetic, nucleic acid sequence, but is not capable of significantly binding, or otherwise hybridizing with, a "natural" or "native" nucleic acid sequence.

Additionally, with respect to the binding pair system employing the synthetic binding unit that includes a 5'-5' joint nucleic acid sequence (that is the nucleic acid binding unit that includes an inverted modification in a segment of the nucleic acid sequence that is relative to the segment that functions as a primer), and the 2' O-Me binding pair system, although these systems may include nucleic acid sequences that may recognize and/or bind to native nucleic acid sequences, these systems both have the advantage that they both employ nucleic acid sequences as primers wherein the nucleic acid sequence includes a modification region such that during the amplification reaction, these modification regions will not be amplified, and thus during detection only the amplified target sequence of interest would be present for immobilization with the synthetic capture unit and detection.

As set forth above, examples of modified nucleic acid sequences include pyranosyl-RNA (pRNA), pyranosyl-DNA (pDNA), 5'-5' inverted DNA, and 2'-O'methylated oligonucleotides, and the like. Further, as set forth above, a chrematistic of the "modified nucleic acids" and the synthetic binding pair systems as disclosed herein is that they all may be employed in diagnostic systems and devices so as to increase the sensitivity of the diagnostic system and/or device in which they are employed. Another characteristic of the pRNA binding pair system is that both the synthetic capture unit and the synthetic binding unit are sterically not capable of pairing with native, e.g., natural, nucleic acids. Additionally, another characteristic of the pRNA binding pair as well as the 2' O-Me systems is that they may not significantly be responsive as substrates of standard nucleic acid enzymes. Other characteristics of the synthetic binding pair systems, as disclosed herein, are that binding of the components of the binding pair system is normally reversible and the position of the equilibrium between free and complexed components of the binding system may be controlled by temperature, pH, concentration and other solution conditions, so as to modulate, e.g., promote, the binding event, or to strip off the associated SBUs from their corresponding SCUs.

In certain instances, a method of the disclosure includes the provision of a surface, such as the surface of a substrate, upon which a synthetic addressable unit may be associated for the detection thereon of an analyte of interest. Any suitable substrate including a surface to which a synthetic addressable complex can be associated may be employed. For instance, the substrate may be formed of glass, such as silicone dioxide; plastic; a mesh network membrane, such as nitrocellulose; and the like. The substrate may be of any suitable shape, such as circular, triangular, square, rectangular, round (e.g., in the shape of a bead), and the like. For example, in certain embodiments, the substrate may be a glass slide, a plastic micro-well plate, a nitrocellulose strip, or the like. In certain embodiments, the substrate is a membrane that is configured for being employed in a lateral flow format. Specifically, in certain embodiments, the substrate may be a nitrocellulose membrane that is configured as a test strip and/or may include one or more of an absorbent pad, e.g., containing a wicking material, and/or a sample pad , e.g., containing an absorbent material.

The surface, for instance, the surface of the substrate, may include a member of the synthetic addressable binding pair system that has been associated with the surface of the substrate. For instance, the surface of the substrate may include a synthetic capture unit (SCU) that has been immobilized at a predetermined location thereon. The member of the synthetic addressable binding pair may be associated with and/or otherwise attached to the surface by any suitable methods, such as those well known in the art (e.g., employing attachment chemistries). In certain embodiments, for instance, where the substrate comprises nitrocellulose, the SCU may be conjugated to a protein, which protein may then be associated with the nitrocellulose substrate, e.g., at a predetermined location. As described above, the SCU is one member of a synthetic addressable binding pair, and as such it is configured for being reversibly coupled to another member of the synthetic addressable binding pair, which other member includes a synthetic binding unit (SBU).

In certain instances, a method of the disclosure includes the contacting of the surface of the substrate containing the associated SCU with the sample, such as a fluid sample obtained from a subject. The sample may be any suitable sample obtained by any suitable means. In certain instances, the sample is a biological sample, such as a sample present in a biological fluid. In such an instance, the biological fluid may be a blood sample, lymph sample, urine sample, saliva sample, excretory sample, tissue sample, cellular sample, and the like. In certain instances, the sample may be obtained from a subject in accordance with extraction methods such as those that are well known in the art. Once obtained the sample may be processed in preparation for detection in accordance with the methods and/or by use of the devices disclosed herein.

For instance, a characteristic of the sample to be contacted with the substrate is that the sample has been preprocessed such that the sample includes the synthetic binding unit (SBU). For example, once the sample has been obtained, e.g., from a biological fluid of a subject, the sample may be processed, in accordance with methods well known in the art, so as to contact the analyte of interest, which is to be detected, with the SBU in a manner sufficient to form an analyte-SBU complex.

In certain exemplary embodiments, e.g., where the analyte to be detected is a nucleic acid, the nucleic acid may be amplified in such a manner so as to produce an analyte-SBU complex, which complex may additionally be labeled. Accordingly, in certain instances, the nucleic acid to be detected may be amplified prior to detection, so as to produce an amplicon. Amplicons of a target nucleic acid to be detected may be generated by any suitable means that is sufficient to produce an amplified product that is associated with an SBU. For instance, in certain embodiments, an amplicon of a nucleic acid to be detected may be generated by use of a thermal cycler employing general protocols, such as polymerase chain reaction (PCR) method, that are well know in the art. For instance, a variety of amplification techniques may be used in a reaction for creating distinguishable amplification products. Some of these techniques employ PCR, as herein disclosed. Other suitable amplification methods include the ligase chain reaction (LCR) (Barringer et al, Gene. 1990, 89(1):117-122.), transcription amplification (Kwoh et al. Proc Natl Acad Sci U S A. 1989 Feb; 86(4):1173-1177; WO88/10315), selective amplification of target polynucleotide sequences (U.S. Pat. No. 6,410,276), consensus sequence primed polymerase chain reaction (U.S. Pat. No. 4,437,975), arbitrarily primed polymerase chain reaction (WO90/06995), nucleic acid based sequence amplification (NASBA) (U.S. Pat. Nos. 5,409,818; 5,554,517; 6,063,603), nick displacement amplification (WO2004/067726).

Accordingly, where the analyte to be detected is a nucleic acid, the nucleic acid may be amplified in a manner sufficient to produce a modified and detectable amplicon, which amplicon is indicative of the presence of the target analyte. Hence, in certain instances, the amplification process of the present disclosure is designed so as to produce a novel amplified product that is recognizable by the SCU and is detectable. To obtain these objectives, in certain instances, the amplification process may employ a pair of modified primers, such as a pair of modified forward and reverse primers. For instance, a first primer, e.g., a forward primer, may be employed wherein the primer is conjugated with the synthetic binding unit (SBU), and a second primer, e.g., a reverse primer, may be employed wherein the primer is labeled or associated with, e.g., conjugated to, a detectable element that itself can be labeled. Methods for the production of a forward nucleic acid primer that is conjugated with a synthetic binding unit are disclosed in US Pub. App. No. 2005/0208576 which is incorporated herein by reference in its entirety.

The first, e.g., forward, primer-SBU conjugate of the disclosure may be utilized in the amplification reaction, such as a polymerase reaction, for the enzymatic amplification of the target nucleic acid to be detected, which may be in the sample or isolated there form. Conjugates comprising at least one synthetic binding unit (SBU) and at least one nucleic acid section (NA) serve as substrate for the enzymatic reaction that utilizes the nucleic acid(s) to be detected as a substrate, and are thus compatible with the commonly used enzymatic processes of biotechnological production methods.

Thus, the basic enzymatic method of the invention is simply contacting a conjugate with at least one enzyme that utilizes naturally occurring nucleic acids as a substrate, and further contacting the mixture with other reagents necessary for the action of the enzyme. Then, the mixture is allowed to incubate under suitable conditions and for a sufficient amount of time for the enzyme to effect the enzymatic modification of the conjugate. Thermostable enzymes, such as those derived from *Thermus aquaticus* and similar species, heat-labile enzymes derived from, e.g., *Escherichia coli* (polymerase, ligase, terminal transferase, etc.) and combinations of enzymes may be utilized in typical amplification processes utilizing concerted enzyme activities, such as TMA (RNA polymerase, RNAse H and reverse transcriptase), and SDA (restriction endonuclease and polymerase).

A second, e.g., a reverse primer, may also be employed in the amplification reaction. For instance, a reverse primer may be employed wherein the primer is conjugated to a detectable moiety. A detectable moiety may itself include a label or may include a member of a binding pair (e.g., biotin) that can bind to its partner (avidin, streptavidin, or the like) which in turn is coupled with a detectable label. Examples of suitable binding pairs in this regard include antigen and antibody, biotin and avidin, carbohydrates and lectins, complementary nucleotide sequences, complementary peptide sequences, effector and receptor molecules, enzyme cofactors and enzymes, enzyme inhibitors and enzymes, a peptide sequence or chemical moiety (such as digoxin anti-digoxin) and an antibody specific for the sequence, chemical moiety or the entire protein, polymeric acids and bases, dyes and protein binders, peptides and specific protein binders (e.g., ribonuclease, S-peptide and ribonuclease S-protein), metals and their chelators, and the like.

A suitable detectable label may be attached to an SBU and/or to a specific binding member, as referenced above, by covalent or non-covalent binding. It is noted that the method of attachment is not critical to the present disclosure. A "label," as used herein, refers to any substance that is capable of producing a signal that is detectable by visual or instrumental means. Various labels suitable for use in the present disclosure include labels that produce signals through either chemical or physical means. Such labels can include enzymes and substrates, chromogens, catalysts, fluorescent or fluorescent like compounds and/or particles, magnetic compounds and/or particles chemiluminescent compounds and or particles, and radioactive labels.

Other suitable labels include particulate labels such as colloidal metallic particles such as gold, colloidal non-metallic particles such as selenium or tellurium, dyed or colored particles such as a dyed plastic or a stained microorganism, organic polymer latex particles and liposomes, colored beads, polymer microcapsules, sacs, erythrocytes, erythrocyte ghosts, or other vesicles containing directly visible substances, and the like. Typically, a visually detectable label is used as the label component of the labeled reagent, thereby providing for the direct visual or instrumental readout of the presence or amount of the analyte in the test sample without the need for additional signal producing components at the detection sites.

Other suitable labels that can be utilized in the practice of the methods include, chromophores, electrochemical moieties, enzymes, radioactive moieties, phosphorescent groups, fluorescent moieties, chemiluminescent moieties, or quantum dots, or more particularly, radiolabels, fluorophore-labels, quantum dot-labels, chromophore-labels, enzyme-labels, affinity ligand-labels, electromagnetic spin labels, heavy atom labels, probes labeled with nanoparticle light scattering labels or other nanoparticles, fluorescein isothiocyanate (FITC), TRITC, rhodamine, tetramethylrhodamine, R-phycoerythrin, Cy-3, Cy-5, Cy-7, molecular beacons and fluorescent derivatives thereof, Texas Red, Phar-Red, allophycocyanin (APC), epitope tags such as the FLAG or HA epitope, and enzyme tags such as alkaline phosphatase, horseradish peroxidase, I²-galactosidase, alkaline phosphatase, β-galactosidase, or acetylcholinesterase and hapten conjugates such as digoxigenin or dinitrophenyl, or members of a binding pair that are capable of forming complexes such as streptavidin/biotin, avidin/biotin or an antigen antibody complex including, for example, rabbit IgG and anti-rabbit IgG; fluorophores such as umbelliferone, fluorescein, fluorescein isothiocyanate, rhodamine, tetramethyl rhodamine, eosin, green fluorescent protein, erythrosin, coumarin, methyl coumarin, pyrene, malachite green, stilbene, lucifer yellow, Cascade Blue, dichlorotriazinylamine fluorescein, dansyl chloride, phycoerythrin, fluorescent lanthanide complexes such as those including europium and terbium; other rare earth elements may also be included such as ytterbium and erbium (see, for instance, Corstjens, P. et al. Clin Chem 2001); a luminescent material such as luminol; light scattering or plasmon resonant materials such as gold or silver particles or quantum dots; or radioactive material include ¹⁴C, ¹²³I, ¹²⁴I, ¹²¹I, ¹³¹I, Tc99m, ³⁵S or ³H, or spherical shells, and probes labeled with any other signal generating label known to those of skill in the art. For example, detectable molecules include but are not limited to fluorophores as well as others known in the art as described, for example, in Principles of Fluorescence Spectroscopy, Joseph R. Lakowicz (Editor), Plenum Pub Corp, 2nd edition (July 1999) and the 6th Edition of the Molecular Probes Handbook by Richard P. Hoagland.

As noted above, in certain instances, labels may include semiconductor nanocrystals, such as quantum dots (Qdots), as described in U.S. Pat. No. 6,207,392 (incorporated in its entirety by reference). Qdots are commercially available from Quantum Dot Corporation. The semiconductor nanocrystals useful in the practice of the invention include nanocrystals of Group II-VI semiconductors such as MgS, MgSe, MgTe, CaS, CaSe, CaTe, SrS, SrSe, SrTe, BaS, BaSe, BaTe, ZnS, ZnSe, ZnTe, CdS, CdSe, CdTe, HgS, HgSe, and HgTe as well as mixed compositions thereof, as well as nanocrystals of Group III-V semiconductors such as GaAs, InGaAs, InP, and InAs and mixed compositions thereof. The use of Group IV semiconductors such as germanium or silicon, or the use of organic semiconductors, may also be feasible under certain conditions. The semiconductor nanocrystals may also include alloys comprising two or more semiconductors selected from the group consisting of the above Group III-V compounds, Group II-VI compounds, Group IV elements, and combinations of same.

In some embodiments, a fluorescent energy acceptor may be linked as a label to a detection probe (e.g., binding moiety conjugated with a detector molecule). In one embodiment the fluorescent energy acceptor may be formed as a result of a compound that reacts with singlet oxygen to form a fluorescent compound or a compound that can react with an auxiliary compound that is thereupon converted to a fluorescent compound. In other embodiments, the fluorescent energy acceptor may be incorporated as part of a compound that also includes the chemiluminescer. For example, the fluorescent energy acceptor may include a metal chelate of a rare earth metal such as, e.g., europium, samarium, tellurium and the like. These materials are particularly attractive because of their sharp band of luminescence. Furthermore, lanthanide labels, such as europium (III) provide for effective and prolonged signal emission and are resistant to photo bleaching.

Long-lifetime fluorescent europium(III) chelate nanoparticles have also been shown to be applicable as labels in various heterogeneous and homogeneous immunoassays. See, e.g., Huhtinen et al., Clin. Chem. 2004 October, 50(10): 1935-6. Assay performance can be improved when these intrinsically labeled nanoparticles are used in combination with time-resolved fluorescence detection. In heterogeneous assays, the dynamic range of assays at low concentrations can be extended. Furthermore, the kinetic characteristics of assays can be improved by use of detection antibody-coated high-specific-activity nanoparticle labels instead of conventionally labeled detection antibodies. In homogeneous assays, europium(III) nanoparticles have been shown to be efficient donors in fluorescence resonance energy transfer, enabling simple and rapid high throughput screening.

In some embodiments, a label (e.g., fluorescent label) disclosed herein, may be configured as a nanoparticle label that is conjugated with a biomolecule. In other words, a nanoparticle can be utilized with a detection or capture probe. For example, a europium(III)-labeled nanoparticle linked to a protein, such as a monoclonal antibody or strepavidin (SA), so as to detect a particular analyte in a sample. Accordingly, in certain instances, a europium(III)-labeled nanoparticle can be utilized in the practice of the present methods (e.g., a nanoparticle-based immunoassay). For instance, in various embodiments of the disclosure, the label utilized may be a lanthanide metal. Lanthanides include but are not limited to europium, samarium, terbium or dysprosium. Non-specific background fluorescence has a decay time of only about 10 ns, so that such background dies away before the sample fluorescence is measured. Furthermore, Lanthanide-chelates have large Stokes' shifts. For example, the Stokes' shift for europium is almost 300 nm. This big difference between excitation and emission peaks means that the fluorescence measurement is made at a wavelength where the influence of background is minimal. In addition, the emission peak is very sharp which means that the detector can be set to very fine limits and that the emission signals from different lanthanide chelates can be easily distinguished from each other. Therefore, in one embodiment, one or more different lanthanides can be utilized in the same assay.

Accordingly, the methods of the disclosure may include the production of an amplicon, such as a labeled amplicon that may be associated with a synthetic binding unit (SBU), and the contacting of the labeled and conjugated amplicon with the surface of the substrate under conditions sufficient for the SBU portion of the amplicon to be recognized and bind to the synthetic capture unit (SCU) fixed or otherwise immobilized on the surface of the substrate such that the amplicon is captured by the SCU and therefore becomes immobilized therewith at the predetermined location, i.e., by the binding of the synthetic capture unit with the synthetic binding unit.

Thus, the amplicon of the nucleic acid to be detected is linked to the synthetic binding unit which is specifically recognized by the complementary synthetic capture unit that is fixed to the surface of the support material. As set forth above, synthetic binding units (SBUs) and synthetic capture units (SCUs) useful in the methods and devices disclosed herein may be molecular units that are capable of a specific molecular pairing. The SBU/SCUs that may be used in the methods discloses herein may include such molecular species as pRNA, pDNA, 5'-5' invert oligonucleotides, or 2'-*O*-methyl oligonucleotides, and the like, such as those disclosed in US Patent No. 5,750,669, which patent is herein incorporated in its entirety by reference.

Hence, the methods disclosed herein may include the contacting of the surface of the substrate with the amplicon under conditions sufficient for the amplicon to become immobilized at the predetermined location by the binding of the synthetic capture unit with the synthetic binding unit. The binding of the synthetic capture unit with the synthetic binding unit produces a synthetic addressable complex that may be labeled, as described above, and is therefore detectable. Once the synthetic capture unit has bound to the synthetic binding unit, the detectable moiety associated with the amplicon may then be detected, by any suitable means known in the art for detecting the associated label, thereby indicating the presence of the nucleic acid in the sample.

### Devices for Detecting the Presence of an Analyte in a Sample

As set forth above, in one aspect, the present disclosure concerns a device for the detection of an analyte in a sample. For instance, in certain embodiments, the present disclosure provides a test device for use in detecting and therefore determining the presence or absence of an analyte, such as an amplicon, in a sample. In general, the test device may include an outside casing and a cavity or lumen and a substrate, such as that described herein below.

The outside casing or housing forms the body of the device and an interior substrate may also be included, wherein the substrate may form a test strip, as described below, that may be present within the cavity of the hosing. The body may be made of any suitable material and may include a sample contacting portion and a read results portion. In certain embodiments, the body in conjunction with the test strip is configured so as to form a matrix. The matrix may define an axial flow path. The matrix may additionally include a sample receiving zone, one or more test zones and optionally, one or more control zones. In certain embodiments, a test region is included, wherein the test region may include the test and control zones, which may be in the form of addressable lines, as described in greater detail herein below.

As used herein in the context of the test device the terms "axial flow membrane", "lateral flow membrane", or "matrix" may be used interchangeably, dependent on the context, which may employ capillary action to move or transport a sample, such as a test fluid, or employs the movement of fluid separate from capillary action as where fluid is pumped by the accumulation of gas pressure, hydraulic pressure (direct pumping using a piston or rotary, bellows or other type pump on the assay fluids, electrostatic movement due to an electric field, gravity, etc.).

In some embodiments, the test strip may include a test membrane substrate. Upstream of the test membrane substrate may be a wicking substrate. Downstream of the test membrane substrate may be disposed another substrate, such as an absorbent substrate. Suitable materials for manufacturing the absorbent substrate includes but are not limited to, hydrophilic polyethylene materials or pads, acrylic fiber, glass fiber, filter paper or pads, desiccated paper, paper pulp, fabric, and the like. For example, the lateral flow membrane absorbent zone may be comprised of a material such as a nonwoven spunlaced acrylic fiber, i.e., New Merge (available from DuPont) or HDK material (available from HDK Industries, Inc.), nonwoven polyethylene treated to improve the hydrophobic property of the material. The test membrane substrate can overlap or abut to one or both the wicking substrate and absorptive substrate, respectively.

For instance, the matrix may include an absorbent zone disposed downstream of the test membrane substrate and a wicking pad can be disposed upstream of the test membrane substrate. In another embodiment, a wicking pad may be disposed directly below a sample entry aperture or opening, as described below. Moreover, in certain embodiments, the test membrane may include a test region, which comprises a test and/or a control zone, that is disposed directly below a window and is thus observable.

Therefore, in accordance with the methods disclosed herein, a test device may be provided, wherein the test device is configured for being used for the receiving of a sample and the detection of an analyte (e.g., one or more amplicons), present in the sample. In certain embodiments, the test device includes a test strip, which test strip may include a lateral flow membrane that is housed within the body of the device. The test device may also include a chamber positioned upstream of the test strip, e.g., lateral flow membrane, which chamber may include a fluid or solution. A gap may also be present, wherein the gap is disposed between the chamber and the test strip, e.g., lateral flow membrane.

The gap may be configured for precluding the fluid communication between the test strip and the chamber. Specifically, in certain embodiments, the housing of the body is configured so as to be depressible and thereby capable of exerting a pressure within the chamber of the device. For instance, in one embodiment, in using the device a pressure may be placed on the outer housing adjacent to the chamber, which pressure pushes close the gap thus forming fluid communication between the chamber and the test strip, e.g., lateral flow membrane, thereby allowing the fluid in the chamber to contact the test strip (e.g., at a test region thereon).

In certain embodiments, the housing may also include an opening. For instance, the housing may include a proximal and a distal end, wherein an opening may be present in the proximal end of the device. The opening may be configured for receiving a sample. Thus, the proximal portion of the device may be termed the sample receiving portion. In certain embodiments, the opening may be configured for contacting a portion of a sample collection device (SCD), which device removably couples to the test device, which coupling allows for the transfer of a sample, contained within the sample collection device, from the SCD to the test device.

For instance, in an optional aspect of the invention, a sample collection device (SCD) may be utilized to collect and/or process a sample with reagents, such as, in certain embodiments, reagents that incorporate a capture moiety and a detection moiety on to the sample. Thus, in various nucleic acid detection systems of the disclosure, an SCD may be provided, wherein the SCD contains one or more chambers comprising reagents for amplification which include a synthetic binding unit, such as a (pRNA), conjugated first primer, a second primer comprising a binding moiety, and DNA polymerase and nucleotides required for an amplification reaction to occur, once the sample is loaded on the SCD. In some embodiments, the SCD may be designed for coupling with a thermal cycler for carrying out a polymerase chain reaction (PCR) thermocycling amplification device. The processed sample from an SCD can then be directly loaded onto the sample receiving zone of a test device.

In embodiments where the test device includes a dipstick configuration, the dipstick can be lowered into the liquid sample of an SCD for loading onto a portion thereof, such as the test zone. Further, the test device can be configured to allow detection of multiple analytes. For instance, such analytes can be from one or more infectious agents, including different strains and/or subtypes thereof. Detection can include qualitative and/or quantitative measurements of one or more analytes. Analytes can also comprise a plurality of analyte types, such as protein and nucleic acid, as described herein below. Where a protein detection is desired, the SCD may include one or more chambers containing immunoreactive reagents that provide a detection means and a capture means for a protein suspected of being present in the sample. Examples of SCDs designed for protein detection are disclosed in US Pub. App. No 2008/0199851, incorporated herein by reference.

Accordingly, in certain embodiments, the opening is positioned above the test strip and is configured such that upon joining with the SCD sample is transferred to the test device from the SCD and contacted with the test strip. For example, in certain embodiments, the opening is configured to receive a distal end of an SCD, which distal end fits into the opening. In certain embodiments, the test strip includes a wicking material which wicking material is positioned below the opening and configured for receiving the sample when transferred. The opening may be disposed directly above a wicking pad that is disposed downstream of the gap, but upstream of the lateral flow membrane.

In some embodiments, a test device includes a plurality of windows. For instance, the body may house a test strip, e.g., a lateral flow membrane, wherein the body further includes one or a plurality of windows through which the test strip, e.g., lateral flow membrane, is viewable. In certain embodiments described herein, the test device includes a lateral flow membrane that comprises a wicking substrate and an absorbent substrate upstream or downstream of test zones disposed on the lateral flow membrane. In some embodiments, the test device may include a plurality of test strips, such as a plurality of parallel test strips, wherein the test strips are positioned within the device in such a manner that when contacted with a sample, the sample flows over all of the test strips, for instance, where it is desired to increase the number of analytes to be detected.

In some embodiments, a substrate for collecting a small volume of sample, e.g., for archiving, is provided in a SCD or test device. In one embodiment, the referenced substrate providing such archiving means is a filter, membrane, or paper that collects a volume of sample. After collection of the sample, the collection substrate may subsequently be removed from the device.

Furthermore, in some embodiments, an SCD and/or test device, as disclosed herein, may include one or more identifiable tags. In certain instances, the identifiable tags are removable and can be removed from one device and placed on another device. For instance, the test device may include an identity label such as a bar code, which identify and correspond to an identical identity label on an SCD and can also identify the lot number of the test device (e.g., for quality assurance and tracking purposes).

As described above, an aspect of the disclosure is a device, such as a test device, that includes a substrate, such as a lateral flow test strip, which substrate may be, though not necessarily need be, encased in a housing. The substrate, e.g., test strip, and/or housing may be designed to be contacted by a sample and read by a reader for the detection of an analyte in the sample.

As used herein, a substrate may be formed as a test strip and refers to the material to which a capture moiety (e.g., an SCU as described above) is linked, e.g., using conventional methods in the art, and to which at least a portion of the sample is contacted. A variety of materials can be used as the substrate, including any material that can act as a support for attachment of the molecules of interest. Such materials are known to those of skill in this art and include, but are not limited to, organic or inorganic polymers, natural and synthetic polymers, including, but not limited to, agarose, cellulose, nitrocellulose, cellulose acetate, other cellulose derivatives, dextran, dextran-derivatives and dextran co-polymers, other polysaccharides, glass, silica gels, gelatin, polyvinyl pyrrolidone (PVP), rayon, nylon, polyethylene, polypropylene, polybutylene, polycarbonate, polyesters, polyamides, vinyl polymers, polyvinylalcohols, polystyrene and polystyrene copolymers, polystyrene cross-linked with divinylbenzene or the like, acrylic resins, acrylates and acrylic acids, acrylamides, polyacrylamide, polyacrylamide blends, co-polymers of vinyl and acrylamide, methacrylates, methacrylate derivatives and co-polymers, other polymers and co-polymers with various functional groups, latex, butyl rubber and other synthetic rubbers, silicon, glass, paper, natural sponges, insoluble protein, surfactants, red blood cells, metals, metalloids, magnetic materials, or other commercially available media or a complex material composed of a solid or semi-solid substrate coated with materials that improve the hydrophilic property of the strip substrate, for example, polystyrene, Mylar, polyethylene, polycarbonate, polypropylene, polybutylene, metals such as aluminum, copper, tin or mixtures of metals coated with dextran, detergents, salts, PVP and/or treated with electrostatic or plasma discharge to add charge to the surface thus imparting a hydrophilic property to the surface.

In one embodiment, the substrate forms a matrix, as described above, wherein the matrix is comprised of a porous material, such as high density polyethylene sheet material manufactured by Porex Technologies Corp. of Fairburn, Ga., USA. The sheet material has an open pore structure with a typical density, at 40% void volume, of 0.57 gm/cc and an average pore diameter of 1 to 250 micrometers, the average generally being from 3 to 100 micrometers. In another embodiment, the substrate is comprised of a porous material such as a nonwoven spunlaced acrylic fiber (similar to the sample receiving zone), e.g., New Merge or HDK material. In certain instances, the porous material may be backed by, or laminated upon, a generally water impervious layer, e.g., Mylar. When employed, the backing is generally fastened to the matrix by an adhesive (e.g., 3M 444 double-sided adhesive tape). Typically, a water impervious backing is used for membranes of low thickness. A wide variety of polymers may be used provided that they do not bind nonspecifically to the assay components and do not interfere with flow of the fluid sample. Illustrative polymers include polyethylene, polypropylene, polystyrene and the like.

In certain embodiments, the substrate forms a matrix, as described above, wherein the matrix may be self-supporting. Other membranes amenable to non-bibulous flow, such as polyvinyl chloride, polyvinyl acetate, copolymers of vinyl acetate and vinyl chloride, polyamide, polycarbonate, polystyrene, and the like, can also be used. In yet another embodiment the matrix forms a lateral flow membrane that is composed of a material such as untreated paper, cellulose blends, nitrocellulose, polyester, an acrylonitrile copolymer, and the like.

As described above, the matrix may include a sample receiving zone and a test zone, wherein the test zone may be constructed to provide either bibulous or non-bibulous flow, frequently the flow type is similar or identical to that provided in at least a portion of the sample receiving zone. In some embodiments, the test zone is comprised of a nonwoven fabric such as Rayon or glass fiber. Other test zone materials suitable for use by the present methods and devices include those chromatographic materials disclosed in U.S. Pat. No. 5,075,078, which is herein incorporated in its entirety by reference.

In one embodiment, the test strip substrate is treated with a solution that includes material-blocking and label-stabilizing agents. Blocking agents include bovine serum albumin (BSA), methylated BSA, casein, acid or base hydrolyzed casein, nonfat dry milk, fish gelatin, or similar. Stabilizing agents are readily available and well known in the art, and may be used, for example, to stabilize labeled reagents.

As set forth above, the substrate may include multiple zones, one of which may be a test zone. A test zone may include one or more lines, which lines contain pre-selected capture moieties (SCU), where a pre-selected region includes capture moieties that are partners for SBUs conjugated to labeled amplicons. In some embodiments, each amplicon is conjugated to a fluorescent label emitting a different wavelength. The SCUs of a given test line may recognize the same SBU as that of a different test line or may recognize a different SBU, as described below. In this manner, a given test device may be used in the testing for and/or detection of one or a plurality of analytes in a sample.

The test region may include one or more zones, such as a test zone and a control zone that are useful to verify that the sample flow is as expected. Each of the control zones may includes a spatially distinct region that may include an immobilized member of a specific binding pair that reacts with a labeled control reagent. In one embodiment, the control zone may contain an authentic sample of the amplicon of interest, or a fragment thereof. In another embodiment, the controlzone may include a line, which line contains antibody that is specific for, or otherwise provides for the immobilization of, the labeled reagent. In operation, a labeled reagent may be restrained in each of the one or more control zones.

The test region may be configured for detecting a single or multiple analytes. Hence, in certain embodiments, a special technical embodiment of the disclosure is a heterogeneous detection device and method, wherein multiple analytes may be detected. Accordingly, a test region of the substrate of the test device may include one or more defined analyte detection zones for the quantitative or qualitative detection of one or more types of analyte in a sample.

For instance, in some embodiments, a synthetic binding unit may be bound to the amplicon and the complementary synthetic capture unit may be immobilized on a line or spot at a predefined location on the substrate. As described above, the synthetic binding unit is specific for a partner or complementary synthetic capture unit (e.g., pRNA specific for complementary pRNA). In one embodiment, the test device includes a combination of different synthetic capture units for the detection of multiple different types of analyte in a sample. Therefore, in various embodiments where two synthetic addressable units are disposed on a substrate, such as a test strip, each will be specific for a different complementary synthetic binding unit.

For example, for two pRNA addressable lines, each line may include pRNA having different sequences which will specifically bind to pRNA of complementary sequences that are themselves bound to different analytes (e.g., an analyte derived from Influenza A versus Influenza B). Thus in one embodiment, a substrate of a test device may be a test strip that includes 1, 2, 3, 4, 5, 6, 7 or 8 addressable test lines.

As set forth above, in one aspect, the present disclosure concerns a system for the detection of an analyte in a sample. For instance, in certain embodiments, the present disclosure concerns a test device for the detection of an analyte in a sample, optionally in conjunction with a sample collection device. The system and methods of the disclose further include the use of a test device, as described herein, in combination with a reader, which reader may be configured for reading a signal from the test device and by detecting the presence or absence of a signal thereon, which signal is indicative of the presence of one or more analytes in a sample being tested, thereby indicating the presence or absence of one or more analytes in a sample being tested.

The reader may be configured to detect a signal generated by a detectable moiety at a site on the test device where an analyte of interest is captured by the synthetic addressable complex. In an embodiment where an amplicon is immobilized on a test strip by the synthetic addressable complex, and wherein the amplicon comprises a biotin-streptavidin conjugated europium label, the reader is configured to determine the presence and location of the amplicon on the test device, by reading the signal generated by the europium label. In some embodiments, the reader is further configured to detect a quantitative amount of signal generated by the immobilized amplicon.

In certain embodiments, the reader may be a reflectance and/or fluorescent based reader, and may include a computer, such as a built in computer. The computer may include data processing software, which software may employ data reduction and curve fitting algorithms, optionally in combination with a trained neural network for accurately determining the presence and/or concentration of analyte in a biological sample. As used herein, a reader refers to an instrument for detecting and/or quantitating data, such as on test strips comprised in a test device. The data may be visible to the naked eye, but does not need to be visible.

Accordingly, the methods employing the systems described herein may include the steps of performing an immunoassay on a patient sample, e.g., using a test device as described herein, reading the data using a reflectance and or fluorescent based reader, and processing the resultant data using data processing software employing data reduction. Exemplary software includes curve fitting algorithms, optionally in combination with a trained neural network, to determine the presence or amount of an analyte, e.g., an amplification product, in a given sample. The data obtained from the reader then can further be processed by a medical diagnosis system program or a person capable of reading the output of the data so as to provide a risk assessment or diagnosis of a medical condition as output. In alternative embodiments, the output can be used as input into a subsequent decision support system, such as a neural network, that is trained to evaluate such data.

As set forth above, the reader is configured for interacting with a substrate containing the synthetic addressable reaction products of the disclosure, which substrate may from part of a test device, as described herein. For instance, in one embodiment, the reader may include a receiving port designed to receive a substrate and/or a test device including a substrate. In certain embodiments, the receiving port is such that the test device may only be inserted into the receiving port if a depressible (e.g., push button) means upstream of a sample entry aperture has been depressed, thereby allowing the test device to properly fit into the receiving port. A reader configured to read a signal generated at the test zone of a test device may also be provided.

In various embodiments, the reader can be a reflectance, transmission, fluorescence, chemo-bioluminescence, magnetic or amperometry reader (or two or more combinations), depending on the signal that is to be detected from the substrate, e.g., of the test device. (e.g., LRE Medical, USA). In one embodiment, the reader is a UV LED reader that detects a fluorescence signal. The fluorescence signal may be excited by a light emitting diode that emits in the UV region of the optics spectrum and within the absorbance peak of the fluorescence signal (e.g., lanthanide label).

The emitted fluorescence signal may be detected by a photodiode and the wavelength of the signal detected may be limited using a long pass filter which blocks stray emitted light and accepts light with wavelengths at and around the peak emission wavelength of the fluorescence emitting label. In other embodiments, the long pass filter may be replaced by a band pass filter. Furthermore, the excitation light may be limited by a band pass filter. In another embodiment, the diode is a UV laser diode. Any conventional UV, LED or photodiode may be utilized. In one embodiment, the reader is adapted with a receiving port for the Test Device.

In one embodiment, the system detects both proteins and nucleic acids in the sample. The detection of proteins is as disclosed in US App. Pub. No. 2008/0199851 incorporated herein by reference. The sample may be prepared for both detection of proteins, by antibody mediated systems using a synthetic binding unit, such as pRNA conjugated to an antibody specific for an antigen; and detection of specific nucleic acids by preparing a SBU-conjugated amplicon as disclosed herein. Both steps may be carried out in a single step. Both steps may be carried out in a sample collection device according to the disclsoure. The prepared sample may then be applied to the test strip, and the presence of both specific nucleic acids and specific proteins in a sample is detected by SBU-SCU mediated binding.

In certain embodiments, the nucleic acid to be detected is not amplified, but rather detected directly. For instance, in certain embodiments, once obtained the sample, e.g., a blood or tissue sample, may be treated with a substance capable of freeing nucleic acids from within the sample, such as nucleic acids of interest to be detected. For example, the sample may be a cell sample and the cell sample may be contacted with a substance, such as a non-ionic detergent, so as to free up the nucleic acids, e.g., DNA or RNA, within the cell in solution.

One or more, e.g., a pair, of oligonucleotides may then be added to the solution, wherein the pair of oligonucleotides include a binding region that is complementary to at least a portion of the freed nucleic acid to be detected, e.g., each member of the pair of oligonucleotides includes a binding region that is complementary to a nucleic acid sequence within the nucleic acid to be detected, wherein the complementary binding regions are different to one another (e.g., the two binding regions are complementary to two different regions of the target nucleic acid to be detected). In this instance, one of the oligonucleotide nucleic acid sequences functions as a binding and the other functions as a labeling unit.

For instance, one of the oligonucleotides may additionally include a sequence, e.g., a binding region, that is complementary to a sequence of a capture unit (e.g., complementary nucleic acid) that is immobilized to a substrate, as described above. Additionally, the other oligonucleotide may include a detection moiety. In this manner, a nucleic acid to be detected may be contacted, e.g., in solution, with both a binding unit and a labeling unit nucleic acid, and may further be contacted with a capture unit nucleic acid immobilized on a substrate, for the immobilization and detection of the nucleic acid sequence of interest. Hence, once the nucleic acid of interest has been immobilized it may then be detected, as described herein, e.g., by detection of the detection moiety.

Additionally, as set forth above, a test device of the disclosure may be configured for detecting both a nucleic acid and a protein in a sample, and in this regard, the nucleic acid and protein may be obtained from the same sample. Hence, where the sample is a tissue sample and includes a virally infected cell, for example, the assay and test device employed may include the detection of two different classes of analytes, such as a nucleic acid and a protein. For instance, a nucleic acid, e.g., a viral RNA or the like, as well as a protein, such as an antibody derived from the subject from whom the sample is obtained, may be detected.

A variety of analytes may be assayed utilizing the method, devices, and systems of the present disclosure. In a particular device useful for assaying for one or more analytes of interest in a sample, the collection of analytes of interest may be referred to as a panel. For example, a panel may comprise any combination analytes, such as nucleic acids and/or proteins, such as antigens and nucleic acids specific for (or all of) of influenza A, influenza B, influenza A subtypes, respiratory syncytial virus (RSV), adenovirus, different types of Parainfluenza viruses (for example Types 1, 2, 3 etc.), HIV variants, Hepatitis A, B and C and other viral agents. Another panel may comprise testing for a selection of one or more of upper respiratory infection including, for example, *Streptococcus pneumoniae, Mycoplasma pneumoniae* and/or *Chlamydia pneumoniae.* Yet another panel can be devised for the diagnosis of sexually transmitted disease including, for example, *Chlamydia, Trichomonas* and/or *Gonorrhea.*

In each case, a particular panel devised to provide signals on the test device for a particular series of analytes may readily be obtained by incorporating a different set of detection and capture probes in the SCD and/or test device described herein.
Therefore, a particular SCD/test device will provide all the reagents necessary to detect one or a particular panel of nucleic acids and/or proteins which are capable of being detected when using a test device employing a substrate, such as a test strip, that has detection reagents that are specific for the analyte(s) of interest.

Thus, a single test device can be used in conjunction with an SCD containing reagents for different panels of analytes, providing enhanced efficiency and cost effectiveness. For example, a panel may optionally include a variety of other analytes of interest, such as a respiratory panel comprising a selection of respiratory agents such as influenza A, influenza B, respiratory syncytial virus, SARS-associated coronavirus, and the like; a hepatitis panel comprising a selection of hepatitis B surface Ag or Ab, hepatitis B core Ab, hepatitis A virus Ab, and hepatitis C virus; a phospholipids panel comprising a selection of Anticardiolipin Abs (IgG, IgA, and IgM Isotypes); an arthritis panel comprising a selection of rheumatoid factor, antinuclear antibodies, and Uric Acid; an Epstein Barr panel comprising a selection of Epstein Barr Nuclear Ag, Epstein Barr Viral Capsid Ag, and Epstein Barr Virus, Early Antigen; other panels include HIV panels, Lupus panels, H. Pylori panels, toxoplasma panels, herpes panels, Borrelia panels, rubella panels, cytomegalovirus panels.

The present disclosure provides kits for use with the methods and systems disclosed herein. A kit may include a test device that includes a substrate, such as a lateral flow strip. The test device may include a test device body and a substrate, such as a lateral flow membrane, positioned in the body and may be exposed through a single or a plurality of windows in the body. The substrate may include an absorbent pad in communication with the lateral flow membrane, the absorbent pad comprising a wicking material and being positioned upstream of said plurality of windows; a sample pad in communication with the lateral flow membrane, the sample pad comprising an absorbent material and being positioned downstream of said plurality of windows; and a plurality of addressable lines, each line having immobilized thereto a synthetic addressable unit, each synthetic addressable unit capable of specifically binding with a synthetic binding unit conjugated to a target analyte.

In some kits a sample collection device, optionally comprising reagents and enzymes for conjugating synthetic binding units and detectable moieties, is provided. The SCD of some kits may be configured to be coupled to the test device for facilitation transfer of reaction mixtures to the test device. Kits may further comprise detectable labels such as streptavidin conjugated europium, and the like. Kits may comprise an instruction manual for operation of the components of the kit in accordance with the present disclosure. Further, a kit may comprise a reader for detection of signal generated in a test device.

### EXAMPLES

Without further elaboration, it is believed that one skilled in the art can, using the preceding description, utilize the present invention to its fullest extent. The following examples are illustrative only, and not limiting of the remainder of the disclosure in any way whatsoever.

### Example 1: Detection using pRNA based synthetic binding system and europium label

In a representative embodiment, the method utilizes pRNA technology and europium beads to detect nucleic acids on a lateral flow strip. The strip consists of three different materials including sample pad, nitrocellulose (NC) membrane and an absorbent pad. One or more unique synthetic nucleotide pRNA polymers (SCU) are coupled to a protein (e.g., immunoglobulin) and then spotted onto NC membranes forming specific test lines. PCR amplicons are generated using a pair of modified primer, of which one primer is conjugated with one type of pRNA (SBU) which is complementary to the pRNA on the NC membrane (SCU) and the other primer is biotinylated. Strips are dipped into samples containing PCR amplicons and streptavidin coated europium beads and incubated at room temperature for 15 min. The presence of amplicons are then detected under UV light on the membrane of the strip through bridging between pRNA and europium beads leading to an accumulation of fluorescent europium beads at the specific test line. Using influenza A as a model target, the detection of limit of the present methods is 0.005ng/µL (=30 amol/µL). This sensitivity is 50-fold greater than NC400 microarray bases tests for influenza A.

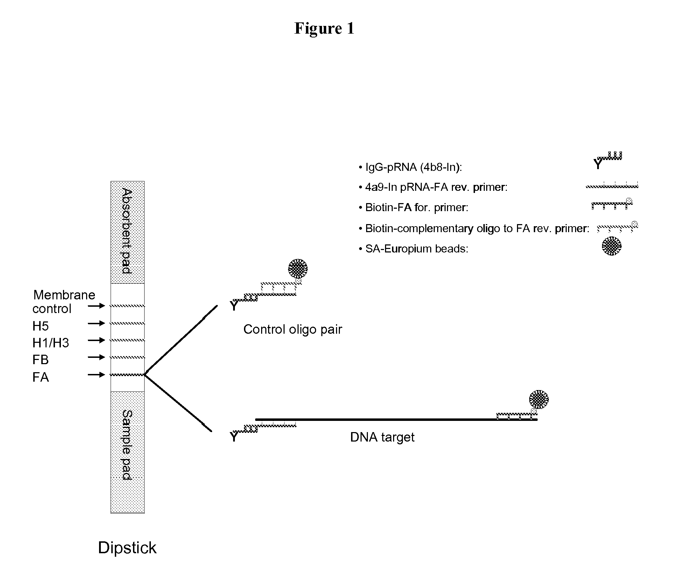

An assay format according to the invention is shown in Figure 1. A test device comprises a test strip in the form of a dipstick. The test strip comprises a sample pad wherein a sample solution of amplicons generated by the methods of the invention are applied. An absorbent pad positioned adjacent to the test pad and distal to the sample pad results in the sample applied to the sample pad traversing through the test pad portion of the test strip. Alternately, the dipstick may be dipped in a solution containing the amplicons. The nitrocellulose (NC) test pad comprises lines of non-specific IgG-pRNA conjugates (4b8-In) immobilized along specific lines designated FA. FA corresponds to a pRNA SCU sequence that is complementary to the pRNA SBU sequence conjugated to a primer for influenza A DNA (4a9-In pRNA-FA reverse primer). Other lines corresponding to FB, H1/H3, H5 and membrane control are also located on the NC test pad.

When an amplicon containing influenza A sequence is generated using a biotinylated forward primer (Biotin-FA) and the 4a9-In pRNA-FA reverse primer, the amplicon attaches to the FA line and is visualized by streptavidin beads conjugated to Europium (SA-Europium beads). The control utilizes a biotinylated complementary oligonucleotide to the FA reverse primer which attaches to the FA IgG-pRNA lines, by directly binding to the reverse primer and binding SA-Europium beads via binding to biotin, as shown in Figure 1.

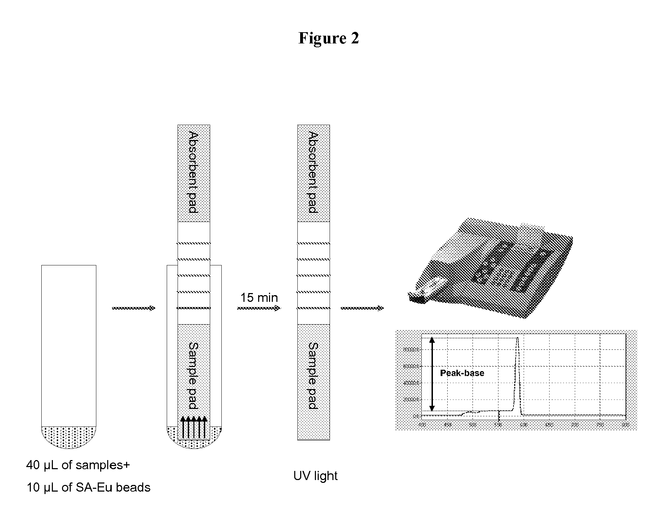

The workflow according to the invention is shown in Figure 2. A typical sample preparation reaction comprises a 40 µl buffered reaction volume wherein an amplification reaction is carried out using a sample (influenza A) DNA, 4a9-In pRNA-FA reverse primer, and biotinylated forward primer (Biotin-FA) and appropriate enzymes, and reagents (NTPs). In addition 10 µL of SA-Eu beads are added for binding to the biotinylated forward primer. In some embodiments, the SA-Eu beads may be added subsequent to the amplification reaction when thermal cycling is used for amplification. Following amplification and binding of the SA-Eu beads, the sample pad portion of the test strip, as described in relation to Figure 1, is dipped in the sample solution.

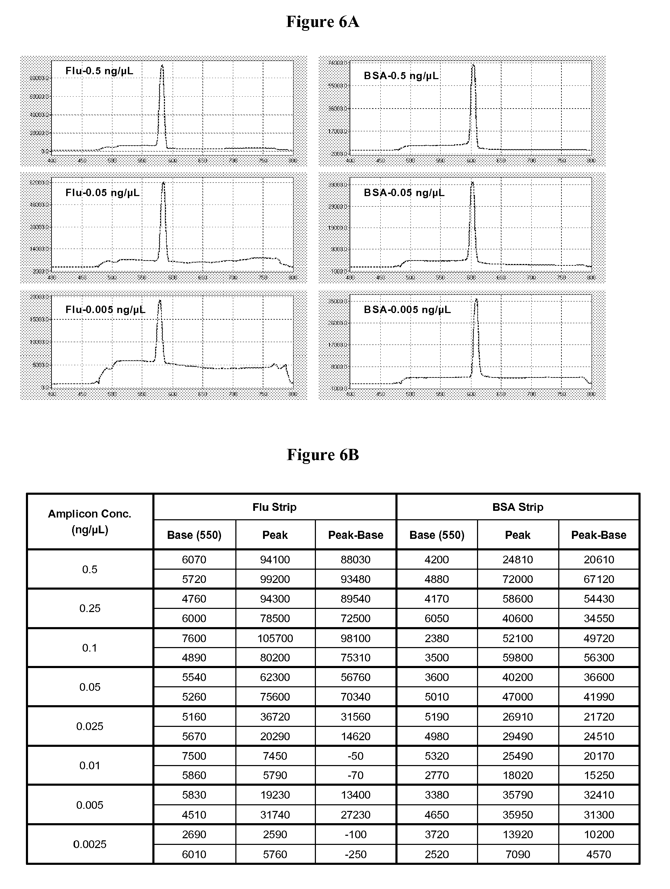

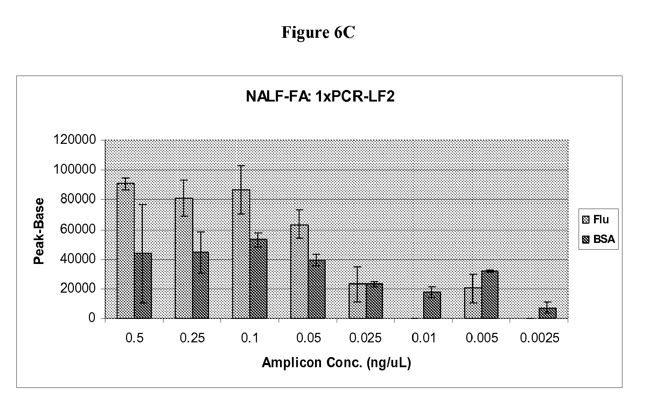

The binding to the addressable SCU lines is completed within a 15 minute incubation period and the bound Eu label is visualized by exposure to UV light. The test strip is scanned by a reader designed to fit an inserted test strip and the location of the label determined as shown in Fig. 6.

The following oligonucleotides and primer mixes were used in the exemplary embodiment:

**Table 1: Oligonucleotide and conjugate primers**

| **Name** | **Function** | **Sequences** |
|---|---|---|
| FA For121477 | Standard fwd primer | 5'-CTT CTA ACC GAG GTC GAA AGG TA-3' |
| FA Rev primer2 | Standard Rev primer | 5'-ACA AAG CGT CTA CGC TGC AGT CC-3' |
| InfAF01 | Biotin-control oligo | 5'-biotin-GGA CTG CAG CGT AGA CGC TTT GT-3' |
| InfAF02 | Biotinylated fwd primer | 5'-biotin-CTT CTA ACC GAG GTC GAA ACG TA-3' |
| 4a9-In pRNA-DNA | pRNA rev primer | pRNA (ATGCDCTTC)-ACA AAG CGT CTA CGC TGC AGT CC-3' |

**Table 2: Primer pairs in each mix**

| | **Mix1 (s/s)** | **Mix2 (bio/pRNA)** | **Mix3 (s/pRNA)** | **Mix4 (bio/s)** | **Control oligo pair** |
|---|---|---|---|---|---|
| **Forward primer** | FA For121477 | InfAF02 | FA For121477 | InfAF02 | InfAF01 |
| **Reverse primer** | FA Rev primer2 | 4a9-In pRNA-DNA | 4a9-In pRNA-DNA | FA Rev primer2 | 4a9-In pRNA-DNA |

The following buffers were used in the exemplary embodiment:

**Table 3: Buffers**

| | **1xPCR Buffer** | **LF Buffer 1** | **Lf Buffer 2** |
|---|---|---|---|
| **Compositions** | 10mM Tris-HCl | 50 mM Tris-HCl | 50 mM Tris-HCl |
| | 50 mM KCl | 3% doc | 0.75 mM NaCl |
| | | 1% casein | 1% BSA |
| | | 1% PEG-8000 | 0.5% digested casein |
| | | pH ∼ 8.0 | 1% saponin |
| | | | 0.25% sulfobetaine-3-12 |
| | | | 0.1% gentamicin solution |
| | | | 0.095% sodium azide |
| | | | 0.03% silicone antifoam |
| | | | 20% sucrose |
| | | | 0.006% FD&C Red 3 |
| | | | pH ∼ 8.5 |
| **Source** | ABI | Sasha Belenky | Nanogen |
| | (58002026-01) | | Wash Buffer with 20% sucrose |
| | | | (601712) |

The following beads were used in the exemplary embodiment:

**Table 4: Beads**

| **Beads 1** 0.2 | **Beads 2** | **Beads 3** |
|---|---|---|
| SA-Eu beads (9.6 mg/mL) → | SA-Eu beads (9.6 mg/mL) → | SA-Eu beads (9.6 mg/mL) → |
| µg/µL in LF buffer 1 | 0.2 µg/µL in LF buffer 2 | 0.2 µg/µL in 1xPCR buffer |

The following test strips were used in the exemplary embodiment:

**Table 5: Test Strips**

| | **IgG Based Strip** | **BSA Bases Strip** |
|---|---|---|
| **Protein conjugated to pRNA** | Non-specific IgG | BSA |
| **Protein concentration (mg/mL)** | 1.5 | 15 |
| **Spot setting (µL/cm)** | 0.75 | 0.5 |
| **Strip width (mm)** | 3.5 | 3.5 |
| **Protein/strip (µg/strip)** | 0.39 | 2.63 |
| **1 µg protein = # of pmols of protein** | 6.7 | 14.9 |
| **Ratio pRNA : protein** | 5 | 7 |
| **pRNA concentration/strip (pmol/strip)** | 13 | 274 |
| **Sample pad** | Yes | No |

### Example 2: Detection Results for the control reaction pair

The control oligonucleotide pair (no amplicon of influenza A DNA) comprising biotinylated complementary oligonucleotide and the 4a9-In pRNA-FA reverse primer which binds to IgG-pRNA were processed as follows:

25 µM of the control oligonucleotide pair was incubated in 1xPCR buffer II at room temperature for ∼45 minutes. The reaction was diluted in LF buffer 1 to 10, 1, 0.5, 0.25, 0.125 and 0.1 pmol/test (pmol/40 µL). SA-Eu beads were diluted in LF buffer 1 to 2 µg/test (2 µg/10µL) and sonicated for 1sec for 4 pulses. 40 µL of the sample and 10µL of SA-Eu beads suspension were transferred to a sample tube. Test strip dipsticks were inserted into each tube and incubated for 15 minutes at room temperature. Each dipstick was checked under UV light for presence of Eu binding. Each dipstick was inserted into a cassette suitable for reading with a fluID^{™} reader and the scan data were analyzed.

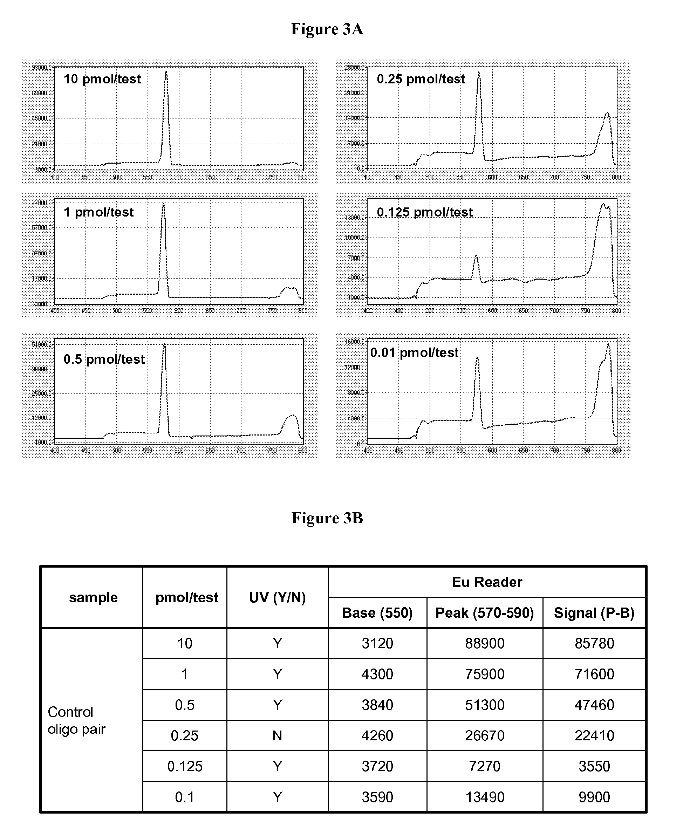

The data from the scan is shown in Figure 3A, and the results summarized in Figure 3B.

### Example 3: Detection Results for FA amplicons

FA amplicons were generated by reverse transcriptase PCR (RT-PCR) reaction with 10, 100 and 1000 copies of transcripts. The amplicon concentration was measured using a capillary electrophoresis system and listed in the following Table.

**Table 6. FA amplicon reactions**

| **FA-transcripts copies/RT-PCR reaction** | **Mix 1(s/s) ng/µL** | **Mix 2 (bio/pRNA) ng/µL** | **Mix 3 (s/pRNA) ng/µL** | **Mix 4 (bio/s) ng/µL** |
|---|---|---|---|---|
| 1000 | 14.94 | 10.27 | 4.75 | 7.2 |
| | 14.31 | 5.09 | 3.98 | 8.02 |
| | 14.43 | 7.51 | 5 | 7.08 |
| 100 | 4.2 | 1.27 | 0.45 | 0.61 |
| | 3.88 | 1.48 | 0.44 | 0.17 |
| | 4.97 | 1.19 | 0.64 | 2.89 |
| 10 | 0 | 0 | 0 | 0 |
| | 0 | 0 | 0 | 0 |
| | 0 | 0 | 0 | 0 |
| NTC | 0 | 0 | 0 | 0 |

FA amplicons were diluted in LF buffer 1 at 1/10, 1/100, and 1/1000 fold. SA-Eu beads were diluted in LF buffer 1 to 2 µg/test (2 µg/10µL) and sonicated for 1 sec for 4 pulses. 40 µL of the sample and 10µL of SA-Eu beads suspension were transferred to a sample tube. Test strip dipsticks were inserted into each tube and incubated for 15 minutes at room temperature. Each dipstick was checked under UV light for presence of Eu binding. Each dipstick was inserted into a cassette suitable for reading with a fluID reader and the scan data were analyzed.

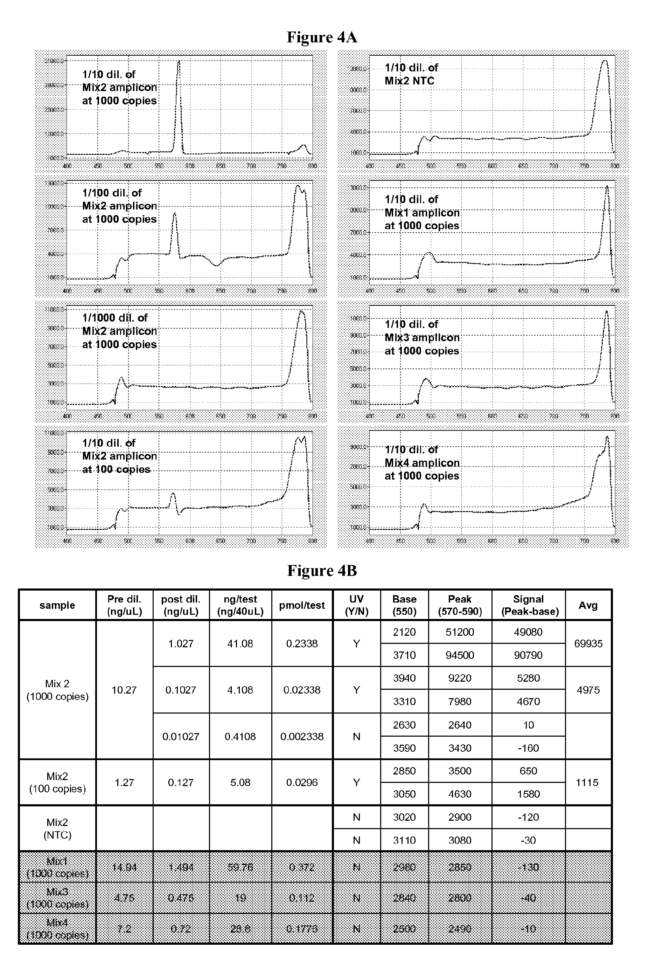

The results are shown in Figures 4A-4B. The scan data is shown in Figure 4A. Signals were detected only on the specific test line where 4b8-In pRNA was spotted. The 4b8-In pRNA is complementary to 4a9-In pRNA. The signals could only be detected for amplicons generated from primer Mix2 that contains of biotinylated InfAf02 and 4a9-In pRNA-DNA as shown in Figure 4B. As expected, no signals were detected for amplicons generated from primer Mix1, Mix3 and Mix4.

### Example 4: Detection Results for FA amplicons with different buffer conditions

FA amplicons with different buffer conditions for amplicons and beads were tested according to the following table.

**Table 7. FA amplicon reactions with different buffer conditions**

| **Combination** | **Buffer for amplicons** | **Buffer for beads** |
|---|---|---|
| 1 (LF1-LF1) | LF1 | LF1 |
| 2 (LF2-LF2) | LF2 | LF2 |
| 3 (1xPCR -- 1xPCR) | 1xPCR | 1xPCR |
| 4 (1xPCR - LF1) | 1xPCR | LF1 |
| 5 (1xPCR - LF2) | 1xPCR | LF2 |
| • Amplicon concentration - 0.5 ng/µL | | |
| • Beads concentration = 0.2 µg/µL | | |
| • Sample volume = 40 µL of amplicon solution + 10 µL of beads suspension | | |

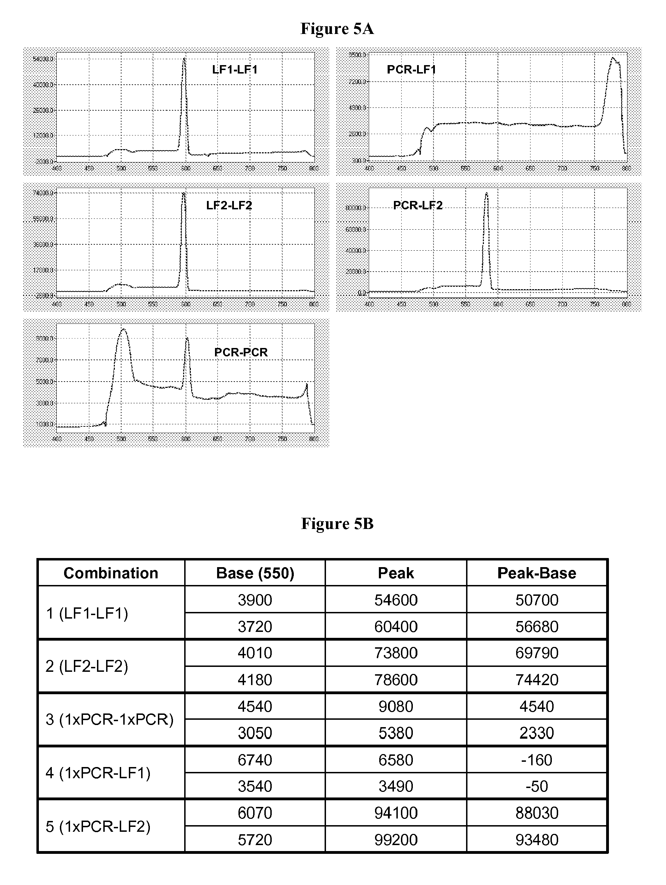

The results of the scan from the reader are shown in Figure 5A and summarized in Figure 5B.

### Example 5: Detection sensitivity for FA amplicons under condition 5

Detection sensitivity for FA amplicons under condition 5 (Amplicon in 1xPCR and SA-Eu beads in LF2) was determined for both IgG-based strip and the BSA-based strip. The results are shown in Figure 6A. The results are summarized in Figure 6B and graphically illustrated in Figure 6C.

Under condition 5, both IgG strip and BSA strip could detect FA amplicons at 0.005 ng/µL, which is 50x more sensitive compared to NC400 (0.25 ng/µL) microarrays. Given that 40 µL of amplicons was used for each strip, the sensitivity is 0.2 ng/strip or 1.2 fmol/test. This sensitivity is comparable those reported in literatures such as a lateral flow chromatograph system (Carter, D.J. and R. B. Cary. Nucleic Acids Research, 2007. 35: e74) or better than a dry reagent dipstick system (Kalogianni, D.P., et al. Nucleic Acids Research, 2007. 35: e23.).

### Example 6: Representative synthetic binding units

Three types of sequences that can function as synthetic binding units are pRNA, 5'-5'invert DNA sequence and 2'-O-methyl oligonucleotides. Typical examples of each are shown below in Table 8.

**Table 8**

| **Type of sequence (in italics)** | **Modified primer (SBD)** | **Complementary oligo on nitrocellulose membrane (SCU)** |
|---|---|---|
| *pRNA* | 5'-(*ATGCDCTTC*)-ACA AAG CGT CTA CGC TGC AGT CC-3' | *GAADGCAT* |
| *5'-5' Invert* | 3'-(*CGC TAT ATG ACG*)-5'-5'-ACA AAG CGT CTA CGC TGC AGT CC-3' | *GCG ATA TAC TGC* |
| *2'-O-methylnucleotides* | 5'-*mUmCmG mCmUmU mGmA*/iSp18/A CAA AGC GTC TAC GCT GCA GTCC-3' | *mUmCmA mAmGmC mGmAmU* |

### Example 7: Synthesis of oligonucleotide-europium complex

Oligonucleotide-europium conjugates are synthesized by mixing the amine-modified oligonucleotide and isothiocyanate-derivatized europium complex under basic conditions at room temperature. A hindered amine base, triethylamine, is utilized in lieu of the sodium bicarbonate buffer to prevent dissociation of the europium from the macrocycle (uncomplexed). Excess metal complex and triethylamine were removed by size exclusion chromatography after completion of the reaction. The oligonucleotide-europium conjugate was separated from un-complexed material by PAGE. Uncomplexed conjugate had a faster gel mobility than the metallated conjugate. Assignments were confirmed by MALDI-TOF mass spectral analyses. BF Baker, et al. Nucleic Acids Research, Vol. 27, Issue 6 1547-1551, (1999).

### Example 8: Europium conjugated Streptavidin

The detection of binding of both proteins and nucleic acid amplicons are based on biotinylated probes detected with europium conjugated Streptavidin (Perkin-Elmer, Boston, MA). The fluorescence of the wells is read with a reader using time-resolved fluorescence settings of 340/612 nm (excitation/emission). *See* Knopf HP, J Immunol Methods. 1991 Apr 25;138(2):233-236.

All publications and patent applications cited in this specification are herein incorporated by reference as if each individual publication or patent application are specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. A method for rapid detection of nucleic acids, the method comprising the steps of:
(a) providing a sample comprising an amplicon of one or more nucleic acid(s) generated using a first primer conjugated to a first synthetic binding unit (SBU) and a second primer conjugated, directly or indirectly, to a first detectable moiety;
(b) providing a surface on a substrate, the surface comprising a first synthetic capture unit (SCU) immobilized at predetermined location on the surface, wherein the first synthetic capture unit (SCU) selectively and reversibly binds the first synthetic binding unit (SBU), but the first synthetic capture unit or the first synthetic binding unit do not participate in the amplification reaction used to generate the amplicon;
(c) contacting the surface with the amplicon under conditions wherein the amplicon is immobilized at the predetermined location by binding between the first synthetic capture unit and the first synthetic binding unit; and
(d) detecting the presence of the nucleic acid by detecting the presence of the first detectable moiety at the predetermined location.

2. The method of claim 1, wherein the surface is a lateral flow membrane comprising a test pad adjacent a sample pad, and the surface is contacted by the amplicons at the sample pad, wherein the test pad and the sample pad are fluidably coupled.

3. The method of claim 1, further comprising:
providing a sample pad adjacent to the surface, wherein a sample contacted with the sample pad is capable of flowing to the surface;
optionally, providing an absorbent pad adjacent to the test pad, wherein the absorbent pad is distal to the sample pad;
applying the sample comprising the amplicon to the sample pad under conditions allowing lateral flow to the surface;
immobilizing the nucleic acid at the predetermined location on the surface by hybridization between the SBU sequence and the SCU sequence immobilized on the surface; and
detecting the presence of the nucleic acid by detecting the presence of the detectable moiety at the predetermined location.

4. The method according to claim 1, for simultaneously detecting a nucleic acid and a protein antigen in a sample, the method further comprising:
(e) forming a mixture by mixing a sample being tested for the presence of at least one target antigen with a solution comprising a plurality of reagents comprising:
(i) an antibody-synthetic binding unit conjugate comprising a first antibody which specifically binds a target antigen and a second synthetic binding unit (SBU) that specifically binds a second synthetic capture unit (SCU), and
(ii) a labeled second antibody, wherein the second antibody specifically binds the same target antigen including when the target antigen is bound to the first antibody, wherein the second antibody is labeled with a second detectable moiety;
(f) applying the mixture and the amplicon solution to the lateral flow membrane comprising a plurality of detection regions, each of said detection region having immobilized thereto a first or second synthetic capture unit;
(g) flowing the mixture and the amplicon solution across the membrane, whereby the first or second synthetic capture unit captures a complex having a first or second synthetic binding unit to which it is directed; and
(h) scanning the membrane for presence of the first and second detectable moieties in at least one of said detection region, whereby detection of the first or second detectable moieties indicates the presence of a target nucleic acid or a target protein in said sample.

5. The method of claim 1, wherein the amplicon is generated by polymerase chain reaction (PCR).

6. The method of claim 1, further comprising: amplifying a nucleic acid using a first primer conjugated to a first SBU, wherein the first SBU does not bind to native nucleic acid.

7. The method of claims 1-4, wherein the detectable moiety is selected from the group consisting of: a fluorophore, a chromophore, a metal, a quantum dot, an enzyme, an electrochemical moieties, a radioactive moiety, a phosphorescent group, a chemiluminescent moiety, an affinity ligand, a heavy atom, a nanoparticle light scattering label, members of a binding pair that are capable of forming complexes comprising streptavidin/biotin, or avidin/biotin, or antigen/antibody, a lanthanide, an europium bead and combinations thereof.

8. The method of claims 1-4, wherein the surface comprises nitrocellulose and the first or second synthetic capture unit is conjugated to a protein.

9. The method of claims 1-4, wherein the synthetic capture unit is immobilized along predetermined test lines on the surface.

10. The method of claims 1-4, wherein the synthetic capture unit is immobilized along predetermined test spots on the surface.

11. A test device for use with the methods of claims 1-4, the test device comprising:
(a) a test device body;
(b) a lateral flow membrane in the body and exposed through a single or a plurality of windows in the body;
(c) an absorbent pad in fluidable communication with the lateral flow membrane, the absorbent pad comprising a wicking material and being positioned upstream of said plurality of windows;
(d) a sample pad in fluidable communication with the lateral flow membrane, the sample pad comprising an absorbent material and being positioned downstream of said plurality of windows; and
(e) a plurality of addressable lines, each line having immobilized thereto a synthetic capture unit, each synthetic capture unit capable of specifically binding with a synthetic binding unit conjugated to a target analyte from a sample.

12. The test device of claim 11, wherein the lateral flow strip is suitable of being scanned by a detection apparatus.

13. The synthetic capture unit and the synthetic binding unit according to claims 1-4 and 11, wherein at least one of the synthetic capture unit and the synthetic binding unit comprise complementary sequences selected from the group consisting of: a pyranosyl RNA (pRNA) sequence, a 2'-O-methyl oligonucleotide sequence, and a 5'-5' inverted nucleic acid sequence.

14. The sample of claims 1-4 and 11, wherein said sample comprises an infectious agent selected from a virus, a bacteria, a fungus and a parasite.

15. The sample of claim 14, wherein the infectious agent is selected from the group consisting of: a strain of influenza virus, parainfluenza virus, a type of HIV, a type of hepatitis virus, a herpes simplex virus, adenovirus, enterovirus, *Streptococcus pneumoniae, Staphylococcus aureus, Bordetella pertussis, Mycoplasma pneumoniae,* and *Coccidioides immitis.*
